# EUROPÄISCHE PATENTANMELDUNG

(11) **EP 2 275 568 A1**
(43) Veröffentlichungstag der Anmeldung: **19.01.2011**
(21) Anmeldenummer: 09165360.0
(22) Anmeldetag: 13.07.2009
(51) Int. Cl.: C12Q 1/68, G01N 33/68

(54) **Markersequenzen für Adipositas und deren Verwendung**

(71) Anmelder: Universitätsklinikum Regensburg, 93053 Regensburg (DE); Protagen AG, 44227 Dortmund (DE)
(72) Erfinder: Schmitz, Gerd, 93161 Sinzing (DE); Hansen, Jens, 59514 Welver-Berwicke (DE); Baessler, Andrea, 93059 Regensburg (DE); Fischer, Marcus, 93059 Regensburg (DE); Lueking, Angelika, 44892 Bochum (DE); Kowald, Axel, 44892 Bochum (DE); Müllner, Stefan, 40764 Langenfeld (DE)
(74) Vertreter: Simandi, Claus

(57) **Zusammenfassung**

Die vorliegende Erfindung betrifft neue Markersequenzen für Adipositas (synonym: Obesitas, engl. obesity (auch: Fettleibigkeit, starkes Übergewicht, Fettsucht)) und deren diagnostische Verwendung samt einem Verfahren zum Screenen von potentiellen Wirkstoffen für Adipositas mittels dieser Markersequenzen. Ferner betrifft die Erfindung eine diagnostische Vorrichtung enthaltend solche Markersequenzen für Adipositas, insbesondere ein Proteinbiochip und dessen Verwendung.

SEQUENCE LISTING

<110>  Protagen Aktiengesellschaft

<120>  Markersequenzen für Obesity

<130>  PRO73DE2009/03

<160>  26

<170>  PatentIn version 3.3

<210>  1
<211>  349
<212>  PRT
<213>  Homo Sapiens

<400>  1

<210>  2
<211>  1076
<212>  PRT
<213>  Homo Sapiens

<400>  2

Met Glu Pro Gly Ser Lys Ser Val Ser Arg Ser Asp Trp Gln Pro Glu
1               5                   10                  15

Pro His Gln Arg Pro Ile Thr Pro Leu Glu Pro Gly Pro Glu Lys Thr
            20                  25                  30

Pro Ile Ala Gln Pro Glu Ser Lys Thr Leu Gln Gly Ser Asn Thr Gln
        35                  40                  45

Gln Lys Pro Ala Ser Asn Gln Arg Pro Leu Thr Gln Gln Glu Thr Pro
    50                  55                  60

Ala Gln His Asp Ala Glu Ser Gln Lys Glu Pro Arg Ala Gln Gln Lys
65                  70                  75                  80

Ser Ala Ser Gln Glu Glu Phe Leu Ala Pro Gln Lys Pro Ala Pro Gln
                85                  90                  95

Gln Ser Pro Tyr Ile Gln Arg Val Leu Leu Thr Gln Gln Glu Ala Ala
            100                 105                 110

Ser Gln Gln Gly Pro Gly Leu Gly Lys Glu Ser Ile Thr Gln Gln Glu
        115                 120                 125

Pro Ala Leu Arg Gln Arg His Val Ala Gln Pro Gly Pro Gly Pro Gly
    130                 135                 140

Glu Pro Pro Pro Ala Gln Gln Glu Ala Glu Ser Thr Pro Ala Ala Gln
145                 150                 155                 160

Ala Lys Pro Gly Ala Lys Arg Glu Pro Ser Ala Pro Thr Glu Ser Thr
                165                 170                 175

Ser Gln Glu Thr Pro Glu Gln Ser Asp Lys Gln Thr Thr Pro Val Gln
            180                 185                 190

Gly Ala Lys Ser Lys Gln Gly Ser Leu Thr Glu Leu Gly Phe Leu Thr
        195                 200                 205

Lys Leu Gln Glu Leu Ser Ile Gln Arg Ser Ala Leu Glu Trp Lys Ala
    210                 215                 220

Leu Ser Glu Trp Val Thr Asp Ser Glu Ser Glu Ser Asp Val Gly Ser
225                 230                 235                 240

Ser Ser Asp Thr Asp Ser Pro Ala Thr Met Gly Gly Met Val Ala Gln
                245                 250                 255

Gly Val Lys Leu Gly Phe Lys Gly Lys Ser Gly Tyr Lys Val Met Ser
            260                 265                 270

Gly Tyr Ser Gly Thr Ser Pro His Glu Lys Thr Ser Ala Arg Asn His
        275                 280                 285

Arg His Tyr Gln Asp Thr Ala Ser Arg Leu Ile His Asn Met Asp Leu
    290                 295                 300

Arg Thr Met Thr Gln Ser Leu Val Thr Leu Ala Glu Asp Asn Ile Ala
305                 310                 315                 320

Phe Phe Ser Ser Gln Gly Pro Gly Glu Thr Ala Gln Arg Leu Ser Gly
                325                 330                 335

Val Phe Ala Gly Val Arg Glu Gln Ala Leu Gly Leu Glu Pro Ala Leu
            340                 345                 350

Gly Arg Leu Leu Gly Val Ala His Leu Phe Asp Leu Asp Pro Glu Thr
        355                 360                 365

Pro Ala Asn Gly Tyr Arg Ser Leu Val His Thr Ala Arg Cys Cys Leu
    370                 375                 380

Ala His Leu Leu His Lys Ser Arg Tyr Val Ala Ser Asn Arg Arg Ser
385                 390                 395                 400

Ile Phe Phe Arg Thr Ser His Asn Leu Ala Glu Leu Glu Ala Tyr Leu
                405                 410                 415

Ala Ala Leu Thr Gln Leu Arg Ala Leu Val Tyr Tyr Ala Gln Arg Leu
            420                 425                 430

Leu Val Thr Asn Arg Pro Gly Val Leu Phe Phe Glu Gly Asp Glu Gly
        435                 440                 445

Leu Thr Ala Asp Phe Leu Arg Glu Tyr Val Thr Leu His Lys Gly Cys
    450                 455                 460

Phe Tyr Gly Arg Cys Leu Gly Phe Gln Phe Thr Pro Ala Ile Arg Pro
465                 470                 475                 480

Phe Leu Gln Thr Ile Ser Ile Gly Leu Val Ser Phe Gly Glu His Tyr
                485                 490                 495

Lys Arg Asn Glu Thr Gly Leu Ser Val Ala Ala Ser Ser Leu Phe Thr
            500                 505                 510

Ser Gly Arg Phe Ala Ile Asp Pro Glu Leu Arg Gly Ala Glu Phe Glu
        515                 520                 525

Arg Ile Thr Gln Asn Leu Asp Val His Phe Trp Lys Ala Phe Trp Asn
    530                 535                 540

Ile Thr Glu Met Glu Val Leu Ser Ser Leu Ala Asn Met Ala Ser Ala
545                 550                 555                 560

Thr Val Arg Val Ser Arg Leu Leu Ser Leu Pro Pro Glu Ala Phe Glu
                565                 570                 575

Met Pro Leu Thr Ala Asp Pro Thr Leu Thr Val Thr Ile Ser Pro Pro
            580                 585                 590

Leu Ala His Thr Gly Pro Gly Pro Val Leu Val Arg Leu Ile Ser Tyr
        595                 600                 605

Asp Leu Arg Glu Gly Gln Asp Ser Glu Glu Leu Ser Ser Leu Ile Lys
    610                 615                 620

Ser Asn Gly Gln Arg Ser Leu Glu Leu Trp Pro Arg Pro Gln Gln Ala
625                 630                 635                 640

Pro Arg Ser Arg Ser Leu Ile Val His Phe His Gly Gly Gly Phe Val
                645                 650                 655

Ala Gln Thr Ser Arg Ser His Glu Pro Tyr Leu Lys Ser Trp Ala Gln
            660                 665                 670

Glu Leu Gly Ala Pro Ile Ile Ser Ile Asp Tyr Ser Leu Ala Pro Glu
        675                 680                 685

Ala Pro Phe Pro Arg Ala Leu Glu Glu Cys Phe Phe Ala Tyr Cys Trp
    690                 695                 700

Ala Ile Lys His Cys Ala Leu Leu Gly Ser Thr Gly Glu Arg Ile Cys
705                 710                 715                 720

Leu Ala Gly Asp Ser Ala Gly Gly Asn Leu Cys Phe Thr Val Ala Leu
                725                 730                 735

Arg Ala Ala Ala Tyr Gly Val Arg Val Pro Asp Gly Ile Met Ala Ala
            740                 745                 750

Tyr Pro Ala Thr Met Leu Gln Pro Ala Ala Ser Pro Ser Arg Leu Leu
        755                 760                 765

Ser Leu Met Asp Pro Leu Leu Pro Leu Ser Val Leu Ser Lys Cys Val
    770                 775                 780

Ser Ala Tyr Ala Gly Ala Lys Thr Glu Asp His Ser Asn Ser Asp Gln
785                 790                 795                 800

Lys Ala Leu Gly Met Met Gly Leu Val Arg Arg Asp Thr Ala Leu Leu
                805                 810                 815

Leu Arg Asp Phe Arg Leu Gly Ala Ser Ser Trp Leu Asn Ser Phe Leu
            820                 825                 830

Glu Leu Ser Gly Arg Lys Ser Gln Lys Met Ser Glu Pro Ile Ala Glu
        835                 840                 845

Pro Met Arg Arg Ser Val Ser Glu Ala Ala Leu Ala Gln Pro Gln Gly
    850                 855                 860

Pro Leu Gly Thr Asp Ser Leu Lys Asn Leu Thr Leu Arg Asp Leu Ser
865                 870                 875                 880

Leu Arg Gly Asn Ser Glu Thr Ser Ser Asp Thr Pro Glu Met Ser Leu
                885                 890                 895

Ser Ala Glu Thr Leu Ser Pro Ser Thr Pro Ser Asp Val Asn Phe Leu
            900                 905                 910

Leu Pro Pro Glu Asp Ala Gly Glu Glu Ala Glu Ala Lys Asn Glu Leu
        915                 920                 925

Ser Pro Met Asp Arg Gly Leu Gly Val Arg Ala Ala Phe Pro Glu Gly
    930                 935                 940

Phe His Pro Arg Arg Ser Ser Gln Gly Ala Thr Gln Met Pro Leu Tyr
945                 950                 955                 960

Ser Ser Pro Ile Val Lys Asn Pro Phe Met Ser Pro Leu Leu Ala Pro
                965                 970                 975

Asp Ser Met Leu Lys Ser Leu Pro Pro Val His Ile Val Ala Cys Ala
            980                 985                 990

Leu Asp Pro Met Leu Asp Asp Ser  Val Met Leu Ala Arg  Arg Leu Arg
        995                 1000                1005

Asn Leu  Gly Gln Pro Val Thr  Leu Arg Val Val Glu  Asp Leu Pro
    1010                1015                1020

His Gly  Phe Leu Thr Leu Ala  Ala Leu Cys Arg Glu  Thr Arg Gln
    1025                1030                1035

Ala Ala  Glu Leu Cys Val Glu  Arg Ile Arg Leu Val  Leu Thr Pro
    1040                1045                1050

Pro Ala  Gly Ala Gly Pro Ser  Gly Glu Thr Gly Ala  Ala Gly Val
    1055                1060                1065

Asp Gly  Gly Cys Gly Gly Arg  His
    1070                1075

<210>  3
<211>  147
<212>  PRT
<213>  Homo Sapiens

<400>  3

Glu Asn Thr Asp Pro Ala Ser Glu Arg Gly Asn Ala Ser Ser Ser Gln
1               5                   10                  15

Lys Glu Lys Val Leu Pro Glu Ala Gly Ala Gly Glu Asp Ser Pro Gly
            20                  25                  30

Arg Asn Met Asp Thr Ala Leu Glu Glu Leu Gln Leu Pro Pro Asn Ala
        35                  40                  45

Glu Gly His Val Lys Gln Val Ser Pro Arg Arg Pro Gln Ala Thr Glu
    50                  55                  60

Ala Val Gly Arg Pro Thr Gly Pro Ala Gly Leu Arg Arg Thr Pro Asn
65                  70                  75                  80

Lys Gln Pro Ser Asp His Ser Tyr Ala Leu Leu Asp Leu Asp Ser Leu
                85                  90                  95

Lys Lys Lys Leu Phe Leu Thr Leu Lys Glu Asn Glu Lys Leu Arg Lys
            100                 105                 110

Arg Leu Gln Ala Gln Arg Leu Val Met Arg Arg Met Ser Ser Arg Leu
        115                 120                 125

Arg Ala Cys Lys Gly His Arg Gly Leu Gln Ala Arg Leu Gly Pro Glu
    130                 135                 140

Gln Gln Ser
145

<210>  4
<211>  264
<212>  PRT
<213>  Homo Sapiens

<400>  4

Met Glu Ala Phe Leu Gly Ser Arg Ser Gly Leu Trp Ala Gly Gly Pro
1               5                   10                  15

Ala Pro Gly Gln Phe Tyr Arg Ile Pro Ser Thr Pro Asp Ser Phe Met
            20                  25                  30

Asp Pro Ala Ser Ala Leu Tyr Arg Gly Pro Ile Thr Arg Thr Gln Asn
        35                  40                  45

Pro Met Val Thr Gly Thr Ser Val Leu Gly Val Lys Phe Glu Gly Gly
    50                  55                  60

Val Val Ile Ala Ala Asp Met Leu Gly Ser Tyr Gly Ser Leu Ala Arg
65                  70                  75                  80

Phe Arg Asn Ile Ser Arg Ile Met Arg Val Asn Asn Ser Thr Met Leu
                85                  90                  95

Gly Ala Ser Gly Asp Tyr Ala Asp Phe Gln Tyr Leu Lys Gln Val Leu
            100                 105                 110

Gly Gln Met Val Ile Asp Glu Glu Leu Leu Gly Asp Gly His Ser Tyr
        115                 120                 125

Ser Pro Arg Ala Ile His Ser Trp Leu Thr Arg Ala Met Tyr Ser Arg
    130                 135                 140

Arg Ser Lys Met Asn Pro Leu Trp Asn Thr Met Val Ile Gly Gly Tyr
145                 150                 155                 160

Ala Asp Gly Glu Ser Phe Leu Gly Tyr Val Asp Met Leu Gly Val Ala
                165                 170                 175

Tyr Glu Ala Pro Ser Leu Ala Thr Gly Tyr Gly Ala Tyr Leu Ala Gln
            180                 185                 190

Pro Leu Leu Arg Glu Val Leu Glu Lys Gln Pro Val Leu Ser Gln Thr
        195                 200                 205

Glu Ala Arg Asp Leu Val Glu Arg Cys Met Arg Val Leu Tyr Tyr Arg
    210                 215                 220

Asp Ala Arg Ser Tyr Asn Arg Phe Gln Ile Ala Thr Val Thr Glu Lys
225                 230                 235                 240

Gly Val Glu Ile Glu Gly Pro Leu Ser Thr Glu Thr Asn Trp Asp Ile
                245                 250                 255

Ala His Met Ile Ser Gly Phe Glu
            260

<210>  5
<211>  249
<212>  PRT
<213>  Homo Sapiens

<400>  5

Met Ala Met Leu Arg Val Gln Pro Glu Ala Gln Ala Lys Val Asp Val
1               5                   10                  15

Phe Arg Glu Asp Leu Cys Thr Lys Thr Glu Asn Leu Leu Gly Ser Tyr
            20                  25                  30

Phe Pro Lys Lys Ile Ser Glu Leu Asp Ala Phe Leu Lys Glu Pro Ala
        35                  40                  45

Leu Asn Glu Ala Asn Leu Ser Asn Leu Lys Ala Pro Leu Asp Ile Pro
    50                  55                  60

Val Pro Asp Pro Val Lys Glu Lys Glu Lys Glu Glu Arg Lys Lys Gln
65                  70                  75                  80

Gln Glu Lys Glu Asp Lys Asp Glu Lys Lys Lys Gly Glu Asp Glu Asp
                85                  90                  95

Lys Gly Pro Pro Cys Gly Pro Val Asn Cys Asn Glu Lys Ile Val Val
            100                 105                 110

Leu Leu Gln Arg Leu Lys Pro Glu Ile Lys Asp Val Ile Glu Gln Leu

        115                 120                 125

Asn Leu Val Thr Thr Trp Leu Gln Leu Gln Ile Pro Arg Ile Glu Asp
    130                 135                 140

Gly Asn Asn Phe Gly Val Ala Val Gln Glu Lys Val Phe Glu Leu Met
145                 150                 155                 160

Thr Ser Leu His Thr Lys Leu Glu Gly Phe His Thr Gln Ile Ser Lys
                165                 170                 175

Tyr Phe Ser Glu Arg Gly Asp Ala Val Thr Lys Ala Ala Lys Gln Pro
            180                 185                 190

His Val Gly Asp Tyr Arg Gln Leu Val His Glu Leu Asp Glu Ala Glu
        195                 200                 205

Tyr Arg Asp Ile Arg Leu Met Val Met Glu Ile Arg Asn Ala Tyr Ala
    210                 215                 220

Val Leu Tyr Asp Ile Ile Leu Lys Asn Phe Glu Lys Leu Lys Lys Pro
225                 230                 235                 240

Arg Gly Glu Thr Lys Gly Met Ile Tyr
                245

<210> 6
<211> 180
<212> PRT
<213> Homo Sapiens

<400> 6

Met Gly Leu Thr Ile Ser Ser Leu Phe Ser Arg Leu Phe Gly Lys Lys
1               5                   10                  15

Gln Met Arg Ile Leu Met Val Gly Leu Asp Ala Ala Gly Lys Thr Thr
            20                  25                  30

Ile Leu Tyr Lys Leu Lys Leu Gly Glu Ile Val Thr Thr Ile Pro Thr
        35                  40                  45

Ile Gly Phe Asn Val Glu Thr Val Glu Tyr Lys Asn Ile Cys Phe Thr
    50                  55                  60

Val Trp Asp Val Gly Gly Gln Asp Arg Ile Arg Pro Leu Trp Lys His
65                  70                  75                  80

Tyr Phe Gln Asn Thr Gln Gly Leu Ile Phe Val Val Asp Ser Asn Asp
                85                  90                  95

Arg Glu Arg Ile Gln Glu Val Ala Asp Glu Leu Gln Lys Met Leu Leu
            100                 105                 110

Val Asp Glu Leu Arg Asp Ala Val Leu Leu Leu Phe Ala Asn Lys Gln
        115                 120                 125

Asp Leu Pro Asn Ala Met Ala Ile Ser Glu Met Thr Asp Lys Leu Gly
    130                 135                 140

Leu Gln Ser Leu Arg Asn Arg Thr Trp Tyr Val Gln Ala Thr Cys Ala
145                 150                 155                 160

Thr Gln Gly Thr Gly Leu Tyr Glu Gly Leu Asp Trp Leu Ser Asn Glu
                165                 170                 175

Leu Ser Lys Arg
            180

<210>  7
<211>  323
<212>  PRT
<213>  Homo Sapiens

<400>  7

Met Ala Pro Lys Gln Asp Pro Lys Pro Lys Phe Gln Glu Gly Glu Arg
1               5                   10                  15

Val Leu Cys Phe His Gly Pro Leu Leu Tyr Glu Ala Lys Cys Val Lys
            20                  25                  30

Val Ala Ile Lys Asp Lys Gln Val Lys Tyr Phe Ile His Tyr Ser Gly
        35                  40                  45

Trp Asn Lys Asn Trp Asp Glu Trp Val Pro Glu Ser Arg Val Leu Lys
    50                  55                  60

Tyr Val Asp Thr Asn Leu Gln Lys Gln Arg Glu Leu Gln Lys Ala Asn
65                  70                  75                  80

Gln Glu Gln Tyr Ala Glu Gly Lys Met Arg Gly Ala Ala Pro Gly Lys
                85                  90                  95

Lys Thr Ser Gly Leu Gln Gln Lys Asn Val Glu Val Lys Thr Lys Lys
            100                 105                 110

Asn Lys Gln Lys Thr Pro Gly Asn Gly Asp Gly Gly Ser Thr Ser Glu
        115                 120                 125

Thr Pro Gln Pro Pro Arg Lys Lys Arg Ala Arg Val Asp Pro Thr Val
    130                 135                 140

Glu Asn Glu Glu Thr Phe Met Asn Arg Val Glu Val Lys Val Lys Ile
145                 150                 155                 160

Pro Glu Glu Leu Lys Pro Trp Leu Val Asp Asp Trp Asp Leu Ile Thr
                165                 170                 175

Arg Gln Lys Gln Leu Phe Tyr Leu Pro Ala Lys Lys Asn Val Asp Ser
            180                 185                 190

Ile Leu Glu Asp Tyr Ala Asn Tyr Lys Lys Ser Arg Gly Asn Thr Asp
        195                 200                 205

Asn Lys Glu Tyr Ala Val Asn Glu Val Val Ala Gly Ile Lys Glu Tyr
    210                 215                 220

Phe Asn Val Met Leu Gly Thr Gln Leu Leu Tyr Lys Phe Glu Arg Pro
225                 230                 235                 240

Gln Tyr Ala Glu Ile Leu Ala Asp His Pro Asp Ala Pro Met Ser Gln
                245                 250                 255

Val Tyr Gly Ala Pro His Leu Leu Arg Leu Phe Val Arg Ile Gly Ala
            260                 265                 270

Met Leu Ala Tyr Thr Pro Leu Asp Glu Lys Ser Leu Ala Leu Leu Leu
        275                 280                 285

Asn Tyr Leu His Asp Phe Leu Lys Tyr Leu Ala Lys Asn Ser Ala Thr
    290                 295                 300

Leu Phe Ser Ala Ser Asp Tyr Glu Val Ala Pro Pro Glu Tyr His Arg
305                 310                 315                 320

Lys Ala Val

<210>  8
<211>  211
<212>  PRT
<213>  Homo Sapiens

<400>  8

Met Cys Pro Gly Asn Trp Leu Trp Ala Ser Met Thr Phe Met Ala Arg
1               5                   10                  15

Phe Ser Arg Ser Ser Ser Arg Ser Pro Val Arg Thr Arg Gly Thr Leu
            20                  25                  30

Glu Glu Met Pro Thr Val Gln His Pro Phe Leu Asn Val Phe Glu Leu
        35                  40                  45

Glu Arg Leu Leu Tyr Thr Gly Lys Thr Ala Cys Asn His Ala Asp Glu
    50                  55                  60

Val Trp Pro Gly Leu Tyr Leu Gly Asp Gln Asp Met Ala Asn Asn Arg
65                  70                  75                  80

Arg Glu Leu Arg Arg Leu Gly Ile Thr His Val Leu Asn Ala Ser His
                85                  90                  95

Ser Arg Trp Arg Gly Thr Pro Glu Ala Tyr Glu Gly Leu Gly Ile Arg
            100                 105                 110

Tyr Leu Gly Val Glu Ala His Asp Ser Pro Ala Phe Asp Met Ser Ile
        115                 120                 125

His Phe Gln Thr Ala Ala Asp Phe Ile His Arg Ala Leu Ser Gln Pro
    130                 135                 140

Gly Gly Lys Ile Leu Val His Cys Ala Val Gly Val Ser Arg Ser Ala
145                 150                 155                 160

Thr Leu Val Leu Ala Tyr Leu Met Leu Tyr His His Leu Thr Leu Val
                165                 170                 175

Glu Ala Ile Lys Lys Val Lys Asp His Arg Gly Ile Ile Pro Asn Arg
            180                 185                 190

Gly Phe Leu Arg Gln Leu Leu Ala Leu Asp Arg Arg Leu Arg Gln Gly
        195                 200                 205

Leu Glu Ala
    210

<210>  9
<211>  180
<212>  PRT
<213>  Homo Sapiens

<400>  9

Met Ser Arg Gln Ala Asn Arg Gly Thr Glu Ser Lys Lys Met Ser Ser
1               5                   10                  15

Glu Leu Phe Thr Leu Thr Tyr Gly Ala Leu Val Thr Gln Leu Cys Lys
            20                  25                  30

Asp Tyr Glu Asn Asp Glu Asp Val Asn Lys Gln Leu Asp Lys Met Gly
        35                  40                  45

Phe Asn Ile Gly Val Arg Leu Ile Glu Asp Phe Leu Ala Arg Ser Asn
    50                  55                  60

Val Gly Arg Cys His Asp Phe Arg Glu Thr Ala Asp Val Ile Ala Lys
65                  70                  75                  80

Val Ala Phe Lys Met Tyr Leu Gly Ile Thr Pro Ser Ile Thr Asn Trp

85                  90                  95

Ser Pro Ala Gly Asp Glu Phe Ser Leu Ile Leu Glu Asn Asn Pro Leu
            100                 105                 110

Val Asp Phe Val Glu Leu Pro Asp Asn His Ser Ser Leu Ile Tyr Ser
        115                 120                 125

Asn Leu Leu Cys Gly Val Leu Arg Gly Ala Leu Glu Met Val Gln Met
    130                 135                 140

Ala Val Glu Ala Lys Phe Val Gln Asp Thr Leu Lys Gly Asp Gly Val
145                 150                 155                 160

Thr Glu Ile Arg Met Arg Phe Ile Arg Arg Ile Glu Asp Asn Leu Pro
                165                 170                 175

Ala Gly Glu Glu
            180

<210>  10
<211>  590
<212>  PRT
<213>  Homo Sapiens

<400>  10

Met Ser Gly Asp Tyr Glu Asp Asp Leu Cys Arg Arg Ala Leu Ile Leu
1               5                   10                  15

Val Ser Asp Leu Cys Ala Arg Val Arg Asp Ala Asp Thr Asn Asp Arg
            20                  25                  30

Cys Gln Glu Phe Asn Asp Arg Ile Arg Gly Tyr Pro Arg Gly Pro Asp
        35                  40                  45

Ala Asp Ile Ser Val Ser Leu Leu Ser Val Ile Val Thr Phe Cys Gly
    50                  55                  60

Ile Val Leu Leu Gly Val Ser Leu Phe Val Ser Trp Lys Leu Cys Trp
65                  70                  75                  80

Val His Trp Arg Asp Lys Gly Gly Ser Ala Val Gly Gly Gly Pro Leu
                85                  90                  95

Arg Lys Asp Leu Gly Pro Gly Val Gly Leu Ala Gly Leu Val Gly Gly
            100                 105                 110

Gly Gly His His Leu Ala Ala Gly Leu Gly Gly His Pro Leu Leu Gly
        115                 120                 125

Gly Pro His His His Ala His Ala Ala His His Pro Pro Phe Ala Glu
    130                 135                 140

Leu Leu Glu Pro Gly Ser Leu Gly Gly Ser Asp Thr Pro Glu Pro Ser
145                 150                 155                 160

Tyr Leu Asp Met Asp Ser Tyr Pro Glu Ala Ala Ala Ala Ala Val Ala
                165                 170                 175

Ala Gly Val Lys Pro Ser Gln Thr Ser Pro Glu Leu Pro Ser Glu Gly
            180                 185                 190

Gly Ala Gly Ser Gly Leu Leu Leu Leu Pro Pro Ser Gly Gly Gly Leu
        195                 200                 205

Pro Ser Ala Gln Ser His Gln Gln Val Thr Ser Leu Ala Pro Thr Thr
    210                 215                 220

Arg Tyr Pro Ala Leu Pro Arg Pro Leu Thr Gln Gln Thr Leu Thr Ser
225                 230                 235                 240

Gln Pro Asp Pro Ser Ser Glu Glu Arg Pro Pro Ala Leu Pro Leu Pro
                245                 250                 255

Leu Pro Gly Gly Glu Glu Lys Ala Lys Leu Ile Gly Gln Ile Lys Pro
            260                 265                 270

Glu Leu Tyr Gln Gly Thr Gly Pro Gly Gly Arg Arg Ser Gly Gly Gly
        275                 280                 285

Pro Gly Ser Gly Glu Ala Gly Thr Gly Ala Pro Cys Gly Arg Ile Ser
    290                 295                 300

Phe Ala Leu Arg Tyr Leu Tyr Gly Ser Asp Gln Leu Val Val Arg Ile
305                 310                 315                 320

Leu Gln Ala Leu Asp Leu Pro Ala Lys Asp Ser Asn Gly Phe Ser Asp
                325                 330                 335

Pro Tyr Val Lys Ile Tyr Leu Leu Pro Asp Arg Lys Lys Lys Phe Gln
            340                 345                 350

Thr Lys Val His Arg Lys Thr Leu Asn Pro Val Phe Asn Glu Thr Phe
        355                 360                 365

Gln Phe Ser Val Pro Leu Ala Glu Leu Ala Gln Arg Lys Leu His Phe
    370                 375                 380

Ser Val Tyr Asp Phe Asp Arg Phe Ser Arg His Asp Leu Ile Gly Gln
385                 390                 395                 400

Val Val Leu Asp Asn Leu Leu Glu Leu Ala Glu Gln Pro Pro Asp Arg
                405                 410                 415

Pro Leu Trp Arg Asp Ile Val Glu Gly Gly Ser Glu Lys Ala Asp Leu
            420                 425                 430

Gly Glu Leu Asn Phe Ser Leu Cys Tyr Leu Pro Thr Ala Gly Arg Leu
        435                 440                 445

Thr Val Thr Ile Ile Lys Ala Ser Asn Leu Lys Ala Met Asp Leu Thr
    450                 455                 460

Gly Phe Ser Asp Pro Tyr Val Lys Ala Ser Leu Ile Ser Glu Gly Arg
465                 470                 475                 480

Arg Leu Lys Lys Arg Lys Thr Ser Ile Lys Lys Asn Thr Leu Asn Pro
                485                 490                 495

Thr Tyr Asn Glu Ala Leu Val Phe Asp Val Ala Pro Glu Ser Val Glu
            500                 505                 510

Asn Val Gly Leu Ser Ile Ala Val Val Asp Tyr Asp Cys Ile Gly His
        515                 520                 525

Asn Glu Val Ile Gly Val Cys Arg Val Gly Pro Asp Ala Ala Asp Pro
    530                 535                 540

His Gly Arg Glu His Trp Ala Glu Met Leu Ala Asn Pro Arg Lys Pro
545                 550                 555                 560

Val Glu His Trp His Gln Leu Val Glu Glu Lys Thr Val Thr Ser Phe
                565                 570                 575

Thr Lys Gly Ser Lys Gly Leu Ser Glu Lys Glu Asn Ser Glu
            580                 585                 590

<210>  11
<211>  490
<212>  PRT
<213>  Homo Sapiens

<400>  11

Met Lys Pro Lys Leu Met Tyr Gln Glu Leu Lys Val Pro Ala Glu Glu
1               5                   10                  15

Pro Ala Asn Glu Leu Pro Met Asn Glu Ile Glu Ala Trp Lys Ala Ala
            20                  25                  30

Glu Lys Lys Ala Arg Trp Val Leu Leu Val Leu Ile Leu Ala Val Val
        35                  40                  45

Gly Phe Gly Ala Leu Met Thr Gln Leu Phe Leu Trp Glu Tyr Gly Asp
    50                  55                  60

Leu His Leu Phe Gly Pro Asn Gln Arg Pro Ala Pro Cys Tyr Asp Pro
65                  70                  75                  80

Cys Glu Ala Val Leu Val Glu Ser Ile Pro Glu Gly Leu Asp Phe Pro
                85                  90                  95

Asn Ala Ser Thr Gly Asn Pro Ser Thr Ser Gln Ala Trp Leu Gly Leu
            100                 105                 110

Leu Ala Gly Ala His Ser Ser Leu Asp Ile Ala Ser Phe Tyr Trp Thr
        115                 120                 125

Leu Thr Asn Asn Asp Thr His Thr Gln Glu Pro Ser Ala Gln Gln Gly
    130                 135                 140

Glu Glu Val Leu Arg Gln Leu Gln Thr Leu Ala Pro Lys Gly Val Asn
145                 150                 155                 160

Val Arg Ile Ala Val Ser Lys Pro Ser Gly Pro Gln Pro Gln Ala Asp
                165                 170                 175

Leu Gln Ala Leu Leu Gln Ser Gly Ala Gln Val Arg Met Val Asp Met
            180                 185                 190

Gln Lys Leu Thr His Gly Val Leu His Thr Lys Phe Trp Val Val Asp
        195                 200                 205

Gln Thr His Phe Tyr Leu Gly Ser Ala Asn Met Asp Trp Arg Ser Leu
    210                 215                 220

Thr Gln Val Lys Glu Leu Gly Val Val Met Tyr Asn Cys Ser Cys Leu
225                 230                 235                 240

Ala Arg Asp Leu Thr Lys Ile Phe Glu Ala Tyr Trp Phe Leu Gly Gln
                245                 250                 255

Ala Gly Ser Ser Ile Pro Ser Thr Trp Pro Arg Phe Tyr Asp Thr Arg
            260                 265                 270

Tyr Asn Gln Glu Thr Pro Met Glu Ile Cys Leu Asn Gly Thr Pro Ala
        275                 280                 285

Leu Ala Tyr Leu Ala Ser Ala Pro Pro Pro Leu Cys Pro Ser Gly Arg
    290                 295                 300

Thr Pro Asp Leu Lys Ala Leu Leu Asn Val Val Asp Asn Ala Arg Ser
305                 310                 315                 320

Phe Ile Tyr Val Ala Val Met Asn Tyr Leu Pro Thr Leu Glu Phe Ser
                325                 330                 335

His Pro His Arg Phe Trp Pro Ala Ile Asp Asp Gly Leu Arg Arg Ala
            340                 345                 350

Thr Tyr Glu Arg Gly Val Lys Val Arg Leu Leu Ile Ser Cys Trp Gly
        355                 360                 365

His Ser Glu Pro Ser Met Arg Ala Phe Leu Leu Ser Leu Ala Ala Leu
    370                 375                 380

Arg Asp Asn His Thr His Ser Asp Ile Gln Val Lys Leu Phe Val Val
385                 390                 395                 400

Pro Ala Asp Glu Ala Gln Ala Arg Ile Pro Tyr Ala Arg Val Asn His
                405                 410                 415

Asn Lys Tyr Met Val Thr Glu Arg Ala Thr Tyr Ile Gly Thr Ser Asn
            420                 425                 430

Trp Ser Gly Asn Tyr Phe Thr Glu Thr Ala Gly Thr Ser Leu Leu Val
        435                 440                 445

Thr Gln Asn Gly Arg Gly Gly Leu Arg Ser Gln Leu Glu Ala Ile Phe
    450                 455                 460

Leu Arg Asp Trp Asp Ser Pro Tyr Ser His Asp Leu Asp Thr Ser Ala
465                 470                 475                 480

Asp Ser Val Gly Asn Ala Cys Arg Leu Leu
                485                 490

<210>  12
<211>  948
<212>  PRT
<213>  Homo Sapiens

<400>  12

Val Tyr Val Pro Ile Leu Pro Ala Ser Leu Leu His Phe Leu Asp Ala
1               5                   10                  15

Pro Val Pro Tyr Leu Met Gly Leu His Ser Asn Gly Leu Asp Asp Arg
            20                  25                  30

Ser Lys Leu Glu Leu Pro Gln Glu Ala Asn Leu Cys Phe Val Asp Ile
        35                  40                  45

Asp Asn His Phe Ile Glu Leu Pro Glu Asp Leu Pro Gln Phe Pro Asn
    50                  55                  60

Lys Leu Glu Phe Val Gln Glu Val Ser Glu Ile Leu Met Ala Phe Gly
65                  70                  75                  80

Ile Pro Pro Glu Gly Asn Leu His Cys Ser Glu Ser Ala Ser Lys Leu
                85                  90                  95

Lys Arg Leu Arg Ala Ser Glu Leu Val Ser Asp Lys Arg Asn Gly Asn
            100                 105                 110

Ile Ala Gly Ser Pro Leu His Ser Tyr Glu Leu Leu Lys Glu Asn Glu
        115                 120                 125

Thr Ile Ala Arg Leu Gln Ala Leu Val Lys Arg Thr Gly Val Ser Leu
    130                 135                 140

Glu Lys Leu Glu Val Arg Glu Asp Pro Ser Ser Asn Lys Asp Leu Lys
145                 150                 155                 160

Val Gln Cys Asp Glu Glu Glu Leu Arg Ile Tyr Gln Leu Asn Ile Gln
                165                 170                 175

Ile Arg Glu Val Phe Ala Asn Arg Phe Thr Gln Met Phe Ala Asp Tyr
            180                 185                 190

Glu Val Phe Val Ile Gln Pro Ser Gln Asp Lys Glu Ser Trp Phe Thr
        195                 200                 205

Asn Arg Glu Gln Met Gln Asn Phe Asp Lys Ala Ser Phe Leu Ser Asp
    210                 215                 220

Gln Pro Glu Pro Tyr Leu Pro Phe Leu Ser Arg Phe Leu Glu Thr Gln
225                 230                 235                 240

Met Phe Ala Ser Phe Ile Asp Asn Lys Ile Met Cys His Asp Asp Asp
                245                 250                 255

Asp Lys Asp Pro Val Leu Arg Val Phe Asp Ser Arg Val Asp Lys Ile
            260                 265                 270

Arg Leu Leu Asn Val Arg Thr Pro Thr Leu Arg Thr Ser Met Tyr Gln
        275                 280                 285

Lys Cys Thr Thr Val Asp Glu Ala Glu Lys Ala Ile Glu Leu Arg Leu
    290                 295                 300

Ala Lys Ile Asp His Thr Ala Ile His Pro His Leu Leu Asp Met Lys
305                 310                 315                 320

Ile Gly Gln Gly Lys Tyr Glu Pro Gly Phe Phe Pro Lys Leu Gln Ser
                325                 330                 335

Asp Val Leu Ser Thr Gly Pro Ala Ser Asn Lys Trp Thr Lys Arg Asn
            340                 345                 350

Ala Pro Ala Gln Trp Arg Arg Lys Asp Arg Gln Lys Gln His Thr Glu
        355                 360                 365

His Leu Arg Leu Asp Asn Asp Gln Arg Glu Lys Tyr Ile Gln Glu Ala
    370                 375                 380

Arg Thr Met Gly Ser Thr Ile Arg Gln Pro Lys Leu Ser Asn Leu Ser
385                 390                 395                 400

Pro Ser Val Ile Ala Gln Thr Asn Trp Lys Phe Val Glu Gly Leu Leu
                405                 410                 415

Lys Glu Cys Arg Asn Lys Thr Lys Arg Met Leu Val Glu Lys Met Gly
            420                 425                 430

Arg Glu Ala Val Glu Leu Gly His Gly Glu Val Asn Ile Thr Gly Val
        435                 440                 445

Glu Glu Asn Thr Leu Ile Ala Ser Leu Cys Asp Leu Leu Glu Arg Ile
    450                 455                 460

Trp Ser His Gly Leu Gln Val Lys Gln Gly Lys Ser Ala Leu Trp Ser
465                 470                 475                 480

His Leu Leu His Tyr Gln Asp Asn Arg Gln Arg Lys Leu Thr Ser Gly
                485                 490                 495

Ser Leu Ser Thr Ser Gly Ile Leu Leu Asp Ser Glu Arg Arg Lys Ser
            500                 505                 510

Asp Ala Ser Ser Leu Met Pro Pro Leu Arg Ile Ser Leu Ile Gln Asp
        515                 520                 525

Met Arg His Ile Gln Asn Ile Gly Glu Ile Lys Thr Asp Val Gly Lys
    530                 535                 540

Ala Arg Ala Trp Val Arg Leu Ser Met Glu Lys Lys Leu Leu Ser Arg
545                 550                 555                 560

His Leu Lys Gln Leu Leu Ser Asp His Glu Leu Thr Lys Lys Leu Tyr
                565                 570                 575

Lys Arg Tyr Ala Phe Leu Arg Cys Asp Asp Glu Lys Glu Gln Phe Leu
            580                 585                 590

Tyr His Leu Leu Ser Phe Asn Ala Val Asp Tyr Phe Cys Phe Thr Asn
        595                 600                 605

Val Phe Thr Thr Ile Leu Ile Pro Tyr His Ile Leu Ile Val Pro Ser
    610                 615                 620

Lys Lys Leu Gly Gly Ser Met Phe Thr Ala Asn Pro Trp Ile Cys Ile
625                 630                 635                 640

Ser Gly Glu Leu Gly Glu Thr Gln Ile Met Gln Ile Pro Arg Asn Val
                645                 650                 655

Leu Glu Met Thr Phe Glu Cys Gln Asn Leu Gly Lys Leu Thr Thr Val
            660                 665                 670

Gln Ile Gly His Asp Asn Ser Gly Leu Tyr Ala Lys Trp Leu Val Glu
        675                 680                 685

Tyr Val Met Val Arg Asn Glu Ile Thr Gly His Thr Tyr Lys Phe Pro
    690                 695                 700

Cys Gly Arg Trp Leu Gly Lys Gly Met Asp Asp Gly Ser Leu Glu Arg
705                 710                 715                 720

Ile Leu Val Gly Glu Leu Leu Thr Ser Gln Pro Glu Val Asp Glu Arg
                725                 730                 735

Pro Cys Arg Thr Pro Pro Leu Gln Gln Ser Pro Ser Val Ile Arg Arg
            740                 745                 750

Leu Val Thr Ile Ser Pro Asn Asn Lys Pro Lys Leu Asn Thr Gly Gln
        755                 760                 765

Ile Gln Glu Ser Ile Gly Glu Ala Val Asn Gly Ile Val Lys His Phe
    770                 775                 780

His Lys Pro Glu Lys Glu Arg Gly Ser Leu Thr Leu Leu Leu Cys Gly
785                 790                 795                 800

Glu Cys Gly Leu Val Ser Ala Leu Glu Gln Ala Phe Gln His Gly Phe
                805                 810                 815

Lys Ser Pro Arg Leu Phe Lys Asn Val Phe Ile Trp Asp Phe Leu Glu
            820                 825                 830

Lys Ala Gln Thr Tyr Tyr Glu Thr Leu Glu Lys Asn Glu Val Val Pro
        835                 840                 845

Glu Glu Asn Trp His Thr Arg Ala Arg Asn Phe Cys Arg Phe Val Thr
    850                 855                 860

Ala Ile Asn Asn Thr Pro Arg Asn Ile Gly Lys Asp Gly Lys Phe Gln
865                 870                 875                 880

Met Leu Val Cys Leu Gly Ala Arg Asp His Leu Leu His His Trp Ile
                885                 890                 895

Ala Leu Leu Ala Asp Cys Pro Ile Thr Ala His Met Tyr Glu Asp Val
            900                 905                 910

Ala Leu Ile Lys Asp His Thr Leu Val Asn Ser Leu Ile Arg Val Leu
        915                 920                 925

Gln Thr Leu Gln Glu Phe Asn Ile Thr Leu Glu Thr Ser Leu Val Lys
    930                 935                 940

Gly Ile Asp Ile
945

<210> 13
<211> 423
<212> PRT
<213> Homo Sapiens

<400> 13

Met Ser Ala Ser Ala Val Tyr Val Leu Asp Leu Lys Gly Lys Val Leu
1               5                   10                  15

Ile Cys Arg Asn Tyr Arg Gly Asp Val Asp Met Ser Glu Val Glu His
            20                  25                  30

Phe Met Pro Ile Leu Met Glu Lys Glu Glu Glu Gly Met Leu Ser Pro
        35                  40                  45

Ile Leu Ala His Gly Gly Val Arg Phe Met Trp Ile Lys His Asn Asn
    50                  55                  60

Leu Tyr Leu Val Ala Thr Ser Lys Lys Asn Ala Cys Val Ser Leu Val
65                  70                  75                  80

Phe Ser Phe Leu Tyr Lys Val Val Gln Val Phe Ser Glu Tyr Phe Lys
                85                  90                  95

Glu Leu Glu Glu Glu Ser Ile Arg Asp Asn Phe Val Ile Ile Tyr Glu
            100                 105                 110

Leu Leu Asp Glu Leu Met Asp Phe Gly Tyr Pro Gln Thr Thr Asp Ser
        115                 120                 125

Lys Ile Leu Gln Glu Tyr Ile Thr Gln Glu Gly His Lys Leu Glu Thr
    130                 135                 140

Gly Ala Pro Arg Pro Pro Ala Thr Val Thr Asn Ala Val Ser Trp Arg
145                 150                 155                 160

Ser Glu Gly Ile Lys Tyr Arg Lys Asn Glu Val Phe Leu Asp Val Ile
                165                 170                 175

Glu Ser Val Asn Leu Leu Val Ser Ala Asn Gly Asn Val Leu Arg Ser

180                 185                 190

Glu Ile Val Gly Ser Ile Lys Met Arg Val Phe Leu Ser Gly Met Pro
        195                 200                 205

Glu Leu Arg Leu Gly Leu Asn Asp Lys Val Leu Phe Asp Asn Thr Gly
    210                 215                 220

Arg Gly Lys Ser Lys Ser Val Glu Leu Glu Asp Val Lys Phe His Gln
225                 230                 235                 240

Cys Val Arg Leu Ser Arg Phe Glu Asn Asp Arg Thr Ile Ser Phe Ile
                245                 250                 255

Pro Pro Asp Gly Glu Phe Glu Leu Met Ser Tyr Arg Leu Asn Thr His
            260                 265                 270

Val Lys Pro Leu Ile Trp Ile Glu Ser Val Ile Glu Lys His Ser His
        275                 280                 285

Ser Arg Ile Glu Tyr Met Ile Lys Ala Lys Ser Gln Phe Lys Arg Arg
    290                 295                 300

Ser Thr Ala Asn Asn Val Glu Ile His Ile Pro Val Pro Asn Asp Ala
305                 310                 315                 320

Asp Ser Pro Lys Phe Lys Thr Thr Val Gly Ser Val Lys Trp Val Pro
                325                 330                 335

Glu Asn Ser Glu Ile Val Trp Ser Ile Lys Ser Phe Pro Gly Gly Lys
            340                 345                 350

Glu Tyr Leu Met Arg Ala His Phe Gly Leu Pro Ser Val Glu Ala Glu
        355                 360                 365

Asp Lys Glu Gly Lys Pro Pro Ile Ser Val Lys Phe Glu Ile Pro Tyr
    370                 375                 380

Phe Thr Thr Ser Gly Ile Gln Val Arg Tyr Leu Lys Ile Ile Glu Lys
385                 390                 395                 400

Ser Gly Tyr Gln Ala Leu Pro Trp Val Arg Tyr Ile Thr Gln Asn Gly
                405                 410                 415

Asp Tyr Gln Leu Arg Thr Gln
            420

<210>  14
<211>  391
<212>  PRT
<213>  Homo Sapiens

<400> 14

Met Ala Asp Ile Asp Asn Lys Glu Gln Ser Glu Leu Asp Gln Asp Leu
1               5                   10                  15

Asp Asp Val Glu Glu Val Glu Glu Glu Glu Thr Gly Glu Glu Thr Lys
            20                  25                  30

Leu Lys Ala Arg Gln Leu Thr Val Gln Met Met Gln Asn Pro Gln Ile
        35                  40                  45

Leu Ala Ala Leu Gln Glu Arg Leu Asp Gly Leu Val Glu Thr Pro Thr
    50                  55                  60

Gly Tyr Ile Glu Ser Leu Pro Arg Val Val Lys Arg Arg Val Asn Ala
65                  70                  75                  80

Leu Lys Asn Leu Gln Val Lys Cys Ala Gln Ile Glu Ala Lys Phe Tyr
                85                  90                  95

Glu Glu Val His Asp Leu Glu Arg Lys Tyr Ala Val Leu Tyr Gln Pro
            100                 105                 110

Leu Phe Asp Lys Arg Phe Glu Ile Ile Asn Ala Ile Tyr Glu Pro Thr
        115                 120                 125

Glu Glu Glu Cys Glu Trp Lys Pro Asp Glu Glu Asp Glu Ile Ser Glu
    130                 135                 140

Glu Leu Lys Glu Lys Ala Lys Ile Glu Asp Glu Lys Lys Asp Glu Glu
145                 150                 155                 160

Lys Glu Asp Pro Lys Gly Ile Pro Glu Phe Trp Leu Thr Val Phe Lys
                165                 170                 175

Asn Val Asp Leu Leu Ser Asp Met Val Gln Glu His Asp Glu Pro Ile
            180                 185                 190

Leu Lys His Leu Lys Asp Ile Lys Val Lys Phe Ser Asp Ala Gly Gln
        195                 200                 205

Pro Met Ser Phe Val Leu Glu Phe His Phe Glu Pro Asn Glu Tyr Phe
    210                 215                 220

Thr Asn Glu Val Leu Thr Lys Thr Tyr Arg Met Arg Ser Glu Pro Asp
225                 230                 235                 240

Asp Ser Asp Pro Phe Ser Phe Asp Gly Pro Glu Ile Met Gly Cys Thr
                245                 250                 255

Gly Cys Gln Ile Asp Trp Lys Lys Gly Lys Asn Val Thr Leu Lys Thr
            260                 265                 270

Ile Lys Lys Lys Gln Lys His Lys Gly Arg Gly Thr Val Arg Thr Val
        275                 280                 285

Thr Lys Thr Val Ser Asn Asp Ser Phe Phe Asn Phe Phe Ala Pro Pro
    290                 295                 300

Glu Val Pro Glu Ser Gly Asp Leu Asp Asp Asp Ala Glu Ala Ile Leu
305                 310                 315                 320

Ala Ala Asp Phe Glu Ile Gly His Phe Leu Arg Glu Arg Ile Ile Pro
                325                 330                 335

Arg Ser Val Leu Tyr Phe Thr Gly Glu Ala Ile Glu Asp Asp Asp Asp
            340                 345                 350

Asp Tyr Asp Glu Glu Gly Glu Glu Ala Asp Glu Glu Gly Glu Glu Glu
        355                 360                 365

Gly Asp Glu Glu Asn Asp Pro Asp Tyr Asp Pro Lys Lys Asp Gln Asn
    370                 375                 380

Pro Ala Glu Cys Lys Gln Gln
385                 390

<210>  15
<211>  295
<212>  PRT
<213>  Homo Sapiens

<400>  15

Ile Pro Asp Gln Ala Arg Val Ala Pro Ser Ser Ser Asp Pro Lys Ser
1               5                   10                  15

Lys Phe Phe Glu Leu Ile Gln Gly Thr Glu Ile Asp Ile Phe Ser Tyr
            20                  25                  30

Lys Pro Thr Leu Leu Thr Ser Lys Thr Leu Glu Lys Ile Arg Pro Val
        35                  40                  45

Phe Asp Tyr Arg Cys Met Val Ser Gly Ser Glu Gln Lys Phe Leu Ile
    50                  55                  60

Gly Leu Gly Lys Ser Gln Ile Tyr Thr Trp Asp Gly Arg Gln Ser Asp
65                  70                  75                  80

Arg Trp Ile Lys Leu Asp Leu Lys Thr Glu Leu Pro Arg Asp Thr Leu
                85                  90                  95

Leu Ser Val Glu Ile Val His Glu Leu Lys Gly Glu Gly Lys Ala Gln
            100                 105                 110

Arg Lys Ile Ser Ala Ile His Ile Leu Asp Val Leu Val Leu Asn Gly
        115                 120                 125

Thr Asp Val Arg Glu Gln His Phe Asn Gln Arg Ile Gln Leu Ala Glu
    130                 135                 140

Lys Phe Val Lys Ala Val Ser Lys Pro Ser Arg Pro Asp Met Asn Pro
145                 150                 155                 160

Ile Arg Val Lys Glu Val Tyr Arg Leu Glu Glu Met Glu Lys Ile Phe
                165                 170                 175

Val Arg Leu Glu Met Lys Ile Ile Lys Gly Ser Ser Gly Thr Pro Lys
            180                 185                 190

Leu Ser Tyr Thr Gly Arg Asp Asp Arg His Phe Val Pro Met Gly Leu
        195                 200                 205

Tyr Ile Val Arg Thr Val Asn Glu Pro Trp Thr Met Gly Phe Ser Lys
    210                 215                 220

Ser Phe Lys Lys Lys Phe Phe Tyr Asn Lys Lys Thr Lys Asp Ser Thr
225                 230                 235                 240

Phe Asp Leu Pro Ala Asp Ser Ile Ala Pro Phe His Ile Cys Tyr Tyr
                245                 250                 255

Gly Arg Leu Phe Trp Glu Trp Gly Asp Gly Ile Arg Val His Asp Ser
            260                 265                 270

Gln Lys Pro Gln Asp Gln Asp Lys Leu Ser Lys Glu Asp Val Leu Ser
        275                 280                 285

Phe Ile Gln Met His Arg Ala
    290                 295

<210>  16
<211>  437
<212>  PRT
<213>  Homo Sapiens

<400>  16

Met Ala Ala Gly Thr Leu Tyr Thr Tyr Pro Glu Asn Trp Arg Ala Phe
1               5                   10                  15

Lys Ala Leu Ile Ala Ala Gln Tyr Ser Gly Ala Gln Val Arg Val Leu
            20                  25                  30

Ser Ala Pro Pro His Phe His Phe Gly Gln Thr Asn Arg Thr Pro Glu
        35                  40                  45

Phe Leu Arg Lys Phe Pro Ala Gly Lys Val Pro Ala Phe Glu Gly Asp
    50                  55                  60

Asp Gly Phe Cys Val Phe Glu Ser Asn Ala Ile Ala Tyr Tyr Val Ser
65                  70                  75                  80

Asn Glu Glu Leu Arg Gly Ser Thr Pro Glu Ala Ala Ala Gln Val Val
                85                  90                  95

Gln Trp Val Ser Phe Ala Asp Ser Asp Ile Val Pro Pro Ala Ser Thr
            100                 105                 110

Trp Val Phe Pro Thr Leu Gly Ile Met His His Asn Lys Gln Ala Thr
        115                 120                 125

Glu Asn Ala Lys Glu Glu Val Arg Arg Ile Leu Gly Leu Leu Asp Ala
    130                 135                 140

Tyr Leu Lys Thr Arg Thr Phe Leu Val Gly Glu Arg Val Thr Leu Ala
145                 150                 155                 160

Asp Ile Thr Val Val Cys Thr Leu Leu Trp Leu Tyr Lys Gln Val Leu
                165                 170                 175

Glu Pro Ser Phe Arg Gln Ala Phe Pro Asn Thr Asn Arg Trp Phe Leu
            180                 185                 190

Thr Cys Ile Asn Gln Pro Gln Phe Arg Ala Val Leu Gly Glu Val Lys
        195                 200                 205

Leu Cys Glu Lys Met Ala Gln Phe Asp Ala Lys Lys Phe Ala Glu Thr
    210                 215                 220

Gln Pro Lys Lys Asp Thr Pro Arg Lys Glu Lys Gly Ser Arg Glu Glu
225                 230                 235                 240

Lys Gln Lys Pro Gln Ala Glu Arg Lys Glu Glu Lys Lys Ala Ala Ala
                245                 250                 255

Pro Ala Pro Glu Glu Glu Met Asp Glu Cys Glu Gln Ala Leu Ala Ala
            260                 265                 270

Glu Pro Lys Ala Lys Asp Pro Phe Ala His Leu Pro Lys Ser Thr Phe
        275                 280                 285

Val Leu Asp Glu Phe Lys Arg Lys Tyr Ser Asn Glu Asp Thr Leu Ser
    290                 295                 300

Val Ala Leu Pro Tyr Phe Trp Glu His Phe Asp Lys Asp Gly Trp Ser
305                 310                 315                 320

Leu Trp Tyr Ser Glu Tyr Arg Phe Pro Glu Glu Leu Thr Gln Thr Phe
                325                 330                 335

Met Ser Cys Asn Leu Ile Thr Gly Met Phe Gln Arg Leu Asp Lys Leu
            340                 345                 350

Arg Lys Asn Ala Phe Ala Ser Val Ile Leu Phe Gly Thr Asn Asn Ser
        355                 360                 365

Ser Ser Ile Ser Gly Val Trp Val Phe Arg Gly Gln Glu Leu Ala Phe
    370                 375                 380

Pro Leu Ser Pro Asp Trp Gln Val Asp Tyr Glu Ser Tyr Thr Trp Arg
385                 390                 395                 400

Lys Leu Asp Pro Gly Ser Glu Glu Thr Gln Thr Leu Val Arg Glu Tyr
                405                 410                 415

Phe Ser Trp Glu Gly Ala Phe Gln His Val Gly Lys Ala Phe Asn Gln
            420                 425                 430

Gly Lys Ile Phe Lys
        435

<210>  17
<211>  514
<212>  PRT
<213>  Homo Sapiens

<400>  17

Met Val Leu Asp Leu Asp Leu Phe Arg Val Asp Lys Gly Gly Asp Pro
1               5                   10                  15

Ala Leu Ile Arg Glu Thr Gln Glu Lys Arg Phe Lys Asp Pro Gly Leu
            20                  25                  30

Val Asp Gln Leu Val Lys Ala Asp Ser Glu Trp Arg Arg Cys Arg Phe
        35                  40                  45

Arg Ala Asp Asn Leu Asn Lys Leu Lys Asn Leu Cys Ser Lys Thr Ile
    50                  55                  60

Gly Glu Lys Met Lys Lys Lys Glu Pro Val Gly Asp Asp Glu Ser Val
65                  70                  75                  80

Pro Glu Asn Val Leu Ser Phe Asp Asp Leu Thr Ala Asp Ala Leu Ala
                85                  90                  95

Asn Leu Lys Val Ser Gln Ile Lys Lys Val Arg Leu Leu Ile Asp Glu
            100                 105                 110

Ala Ile Leu Lys Cys Asp Ala Glu Arg Ile Lys Leu Glu Ala Glu Arg

        115                 120                 125

Phe Glu Asn Leu Arg Glu Ile Gly Asn Leu Leu His Pro Ser Val Pro
    130                 135                 140

Ile Ser Asn Asp Glu Asp Val Asp Asn Lys Val Glu Arg Ile Trp Gly
145                 150                 155                 160

Asp Cys Thr Val Arg Lys Lys Tyr Ser His Val Asp Leu Val Val Met
                165                 170                 175

Val Asp Gly Phe Glu Gly Glu Lys Gly Ala Val Val Ala Gly Ser Arg
            180                 185                 190

Gly Tyr Phe Leu Lys Gly Val Leu Val Phe Leu Glu Gln Ala Leu Ile
        195                 200                 205

Gln Tyr Ala Leu Arg Thr Leu Gly Ser Arg Gly Tyr Ile Pro Ile Tyr
    210                 215                 220

Thr Pro Phe Phe Met Arg Lys Glu Val Met Gln Glu Val Ala Gln Leu
225                 230                 235                 240

Ser Gln Phe Asp Glu Glu Leu Tyr Lys Val Ile Gly Lys Gly Ser Glu
                245                 250                 255

Lys Ser Asp Asp Asn Ser Tyr Asp Glu Lys Tyr Leu Ile Ala Thr Ser
            260                 265                 270

Glu Gln Pro Ile Ala Ala Leu His Arg Asp Glu Trp Leu Arg Pro Glu
        275                 280                 285

Asp Leu Pro Ile Lys Tyr Ala Gly Leu Ser Thr Cys Phe Arg Gln Glu
    290                 295                 300

Val Gly Ser His Gly Arg Asp Thr Arg Gly Ile Phe Arg Val His Gln
305                 310                 315                 320

Phe Glu Lys Ile Glu Gln Phe Val Tyr Ser Ser Pro His Asp Asn Lys
                325                 330                 335

Ser Trp Glu Met Phe Glu Glu Met Ile Thr Thr Ala Glu Glu Phe Tyr
            340                 345                 350

Gln Ser Leu Gly Ile Pro Tyr His Ile Val Asn Ile Val Ser Gly Ser
        355                 360                 365

Leu Asn His Ala Ala Ser Lys Lys Leu Asp Leu Glu Ala Trp Phe Pro
    370                 375                 380

Gly Ser Gly Ala Phe Arg Glu Leu Val Ser Cys Ser Asn Cys Thr Asp

385                 390                 395                 400

Tyr Gln Ala Arg Arg Leu Arg Ile Arg Tyr Gly Gln Thr Lys Lys Met
                405                 410                 415

Met Asp Lys Val Glu Phe Val His Met Leu Asn Ala Thr Met Cys Ala
            420                 425                 430

Thr Thr Cys Thr Ile Cys Ala Ile Leu Glu Asn Tyr Gln Thr Glu Lys
        435                 440                 445

Gly Ile Thr Val Pro Glu Lys Leu Lys Glu Phe Met Pro Pro Gly Leu
    450                 455                 460

Gln Glu Leu Ile Pro Phe Val Lys Pro Ala Pro Ile Glu Gln Glu Pro
465                 470                 475                 480

Ser Lys Lys Gln Lys Lys Gln His Glu Gly Ser Lys Lys Lys Ala Ala
                485                 490                 495

Ala Arg Asp Val Thr Leu Glu Asn Arg Leu Gln Asn Met Glu Val Thr
            500                 505                 510

Asp Ala


<210>  18
<211>  318
<212>  PRT
<213>  Homo Sapiens

<400>  18

Met Ala Ser Pro Gly Cys Leu Leu Cys Val Leu Gly Leu Leu Leu Cys
1               5                   10                  15

Gly Ala Ala Ser Leu Glu Leu Ser Arg Pro His Gly Asp Thr Ala Lys
            20                  25                  30

Lys Pro Ile Ile Gly Ile Leu Met Gln Lys Cys Arg Asn Lys Val Met
        35                  40                  45

Lys Asn Tyr Gly Arg Tyr Tyr Ile Ala Ala Ser Tyr Val Lys Tyr Leu
    50                  55                  60

Glu Ser Ala Gly Ala Arg Val Val Pro Val Arg Leu Asp Leu Thr Glu
65                  70                  75                  80

Lys Asp Tyr Glu Ile Leu Phe Lys Ser Ile Asn Gly Ile Leu Phe Pro
                85                  90                  95

Gly Gly Ser Val Asp Leu Arg Arg Ser Asp Tyr Ala Lys Val Ala Lys
            100                 105                 110

Ile Phe Tyr Asn Leu Ser Ile Gln Ser Phe Asp Asp Gly Asp Tyr Phe
        115                 120                 125

Pro Val Trp Gly Thr Cys Leu Gly Phe Glu Glu Leu Ser Leu Leu Ile
    130                 135                 140

Ser Gly Glu Cys Leu Leu Thr Ala Thr Asp Thr Val Asp Val Ala Met
145                 150                 155                 160

Pro Leu Asn Phe Thr Gly Gly Gln Leu His Ser Arg Met Phe Gln Asn
                165                 170                 175

Phe Pro Thr Glu Leu Leu Leu Ser Leu Ala Val Glu Pro Leu Thr Ala
            180                 185                 190

Asn Phe His Lys Trp Ser Leu Ser Val Lys Asn Phe Thr Met Asn Glu
        195                 200                 205

Lys Leu Lys Lys Phe Phe Asn Val Leu Thr Thr Asn Thr Asp Gly Lys
    210                 215                 220

Ile Glu Phe Ile Ser Thr Met Glu Gly Tyr Lys Tyr Pro Val Tyr Gly
225                 230                 235                 240

Val Gln Trp His Pro Glu Lys Ala Pro Tyr Glu Trp Lys Asn Leu Asp
                245                 250                 255

Gly Ile Ser His Ala Pro Asn Ala Val Lys Thr Ala Phe Tyr Leu Ala
            260                 265                 270

Glu Phe Phe Val Asn Glu Ala Arg Lys Asn Asn His His Phe Lys Ser
        275                 280                 285

Glu Ser Glu Glu Glu Lys Ala Leu Ile Tyr Gln Phe Ser Pro Ile Tyr
    290                 295                 300

Thr Gly Asn Ile Ser Ser Phe Gln Gln Cys Tyr Ile Phe Asp
305                 310                 315

<210>  19
<211>  333
<212>  PRT
<213>  Homo Sapiens

<400>  19

Ala Thr Leu Lys Glu Lys Leu Ile Ala Pro Val Ala Glu Glu Glu Ala
1               5                   10                  15

Thr Val Pro Asn Asn Lys Ile Thr Val Val Gly Val Gly Gln Val Gly
            20                  25                  30

Met Ala Cys Ala Ile Ser Ile Leu Gly Lys Ser Leu Ala Asp Glu Leu
        35                  40                  45

Ala Leu Val Asp Val Leu Glu Asp Lys Leu Lys Gly Glu Met Met Asp
    50                  55                  60

Leu Gln His Gly Ser Leu Phe Leu Gln Thr Pro Lys Ile Val Ala Asp
65                  70                  75                  80

Lys Asp Tyr Ser Val Thr Ala Asn Ser Lys Ile Val Val Val Thr Ala
                85                  90                  95

Gly Val Arg Gln Gln Glu Gly Glu Ser Arg Leu Asn Leu Val Gln Arg
            100                 105                 110

Asn Val Asn Val Phe Lys Phe Ile Ile Pro Gln Ile Val Lys Tyr Ser
        115                 120                 125

Pro Asp Cys Ile Ile Ile Val Val Ser Asn Pro Val Asp Ile Leu Thr
    130                 135                 140

Tyr Val Thr Trp Lys Leu Ser Gly Leu Pro Lys His Arg Val Ile Gly
145                 150                 155                 160

Ser Gly Cys Asn Leu Asp Ser Ala Arg Phe Arg Tyr Leu Met Ala Glu
                165                 170                 175

Lys Leu Gly Ile His Pro Ser Ser Cys His Gly Trp Ile Leu Gly Glu
            180                 185                 190

His Gly Asp Ser Ser Val Ala Val Trp Ser Gly Val Asn Val Ala Gly
        195                 200                 205

Val Ser Leu Gln Glu Leu Asn Pro Glu Met Gly Thr Asp Asn Asp Ser
    210                 215                 220

Glu Asn Trp Lys Glu Val His Lys Met Val Val Glu Ser Ala Tyr Glu
225                 230                 235                 240

Val Ile Lys Leu Lys Gly Tyr Thr Asn Trp Ala Ile Gly Leu Ser Val
                245                 250                 255

Ala Asp Leu Ile Glu Ser Met Leu Lys Asn Leu Ser Arg Ile His Pro
            260                 265                 270

Val Ser Thr Met Val Lys Gly Met Tyr Gly Ile Glu Asn Glu Val Phe
        275                 280                 285

Leu Ser Leu Pro Cys Ile Leu Asn Ala Arg Gly Leu Thr Ser Val Ile
    290                 295                 300

Asn Gln Lys Leu Lys Asp Asp Glu Val Ala Gln Leu Lys Lys Ser Ala
305                 310                 315                 320

Asp Thr Leu Trp Asp Ile Gln Lys Asp Leu Lys Phe Leu
                325                 330

<210>  20
<211>  321
<212>  PRT
<213>  Homo Sapiens

<400>  20

Met Gly Glu Pro Gln Gly Ser Met Arg Ile Leu Val Thr Gly Gly Ser
1               5                   10                  15

Gly Leu Val Gly Lys Ala Ile Gln Lys Val Val Ala Asp Gly Ala Gly
            20                  25                  30

Leu Pro Gly Glu Asp Trp Val Phe Val Ser Ser Lys Asp Ala Asp Leu
        35                  40                  45

Thr Asp Thr Ala Gln Thr Arg Ala Leu Phe Glu Lys Val Gln Pro Thr
    50                  55                  60

His Val Ile His Leu Ala Ala Met Val Gly Gly Leu Phe Arg Asn Ile
65                  70                  75                  80

Lys Tyr Asn Leu Asp Phe Trp Arg Lys Asn Val His Met Asn Asp Asn
                85                  90                  95

Val Leu His Ser Ala Phe Glu Val Gly Ala Arg Lys Val Val Ser Cys
            100                 105                 110

Leu Ser Thr Cys Ile Phe Pro Asp Lys Thr Thr Tyr Pro Ile Asp Glu
        115                 120                 125

Thr Met Ile His Asn Gly Pro Pro His Asn Ser Asn Phe Gly Tyr Ser
    130                 135                 140

Tyr Ala Lys Arg Met Ile Asp Val Gln Asn Arg Ala Tyr Phe Gln Gln
145                 150                 155                 160

Tyr Gly Cys Thr Phe Thr Ala Val Ile Pro Thr Asn Val Phe Gly Pro
                165                 170                 175

His Asp Asn Phe Asn Ile Glu Asp Gly His Val Leu Pro Gly Leu Ile
            180                 185                 190

His Lys Val His Leu Ala Lys Ser Ser Gly Ser Ala Leu Thr Val Trp
        195                 200                 205

Gly Thr Gly Asn Pro Arg Arg Gln Phe Ile Tyr Ser Leu Asp Leu Ala
    210                 215                 220

Gln Leu Phe Ile Trp Val Leu Arg Glu Tyr Asn Glu Val Glu Pro Ile
225                 230                 235                 240

Ile Leu Ser Val Gly Glu Glu Asp Glu Val Ser Ile Lys Glu Ala Ala
                245                 250                 255

Glu Ala Val Val Glu Ala Met Asp Phe His Gly Glu Val Thr Phe Asp
            260                 265                 270

Thr Thr Lys Ser Asp Gly Gln Phe Lys Lys Thr Ala Ser Asn Ser Lys
        275                 280                 285

Leu Arg Thr Tyr Leu Pro Asp Phe Arg Phe Thr Pro Phe Lys Gln Ala
    290                 295                 300

Val Lys Glu Thr Cys Ala Trp Phe Thr Asp Asn Tyr Glu Gln Ala Arg
305                 310                 315                 320

Lys

<210>  21
<211>  734
<212>  PRT
<213>  Homo Sapiens

<400>  21

<210>  22
<211>  694
<212>  PRT
<213>  Homo Sapiens

<400>  22

Ile Leu Thr Gly Ala His Arg Phe Thr Leu Ser Ala Asn Val Gly Asp
1               5                   10                  15

Leu Lys Leu Arg Arg Met Pro Glu Val Pro Gly Leu Gln Ser Ala Pro
            20                  25                  30

Ser Cys Trp Thr Val Leu Cys Gln Asp Arg Val Ala His Ile Leu Leu
        35                  40                  45

Ala Val Gly Pro Asp Leu Tyr Leu Leu Asp His Ala Ala Cys Ser Ala
    50                  55                  60

Val Thr Pro Pro Gly Leu Ala Pro Gly Val Ser Ser Phe Leu Gln Met
65                  70                  75                  80

Ala Val Ser Phe Thr Tyr Arg His Leu Ala Leu Phe Thr Asp Thr Gly
                85                  90                  95

Tyr Ile Trp Met Gly Thr Ala Ser Leu Lys Glu Lys Leu Cys Glu Phe
            100                 105                 110

Asn Cys Asn Ile Arg Ala Pro Pro Lys Gln Met Val Trp Cys Ser Arg
        115                 120                 125

Pro Arg Ser Lys Glu Arg Ala Val Val Val Ala Trp Glu Arg Arg Leu
    130                 135                 140

Met Val Val Gly Asp Ala Pro Glu Ser Ile Gln Phe Val Leu Asp Glu
145                 150                 155                 160

Asp Ser Tyr Leu Val Pro Glu Leu Asp Gly Val Arg Ile Phe Ser Arg
                165                 170                 175

Ser Thr His Glu Phe Leu His Glu Val Pro Ala Ala Ser Glu Glu Ile
            180                 185                 190

Phe Lys Ile Ala Ser Met Ala Pro Gly Ala Leu Leu Leu Glu Ala Gln
        195                 200                 205

Lys Glu Tyr Glu Lys Glu Ser Gln Lys Ala Asp Glu Tyr Leu Arg Glu
    210                 215                 220

Ile Gln Glu Leu Gly Gln Leu Thr Gln Ala Val Gln Gln Cys Ile Glu
225                 230                 235                 240

Ala Ala Gly His Glu His Gln Pro Asp Met Gln Lys Ser Leu Leu Arg
                245                 250                 255

Ala Ala Ser Phe Gly Lys Cys Phe Leu Asp Arg Phe Pro Pro Asp Ser
            260                 265                 270

Phe Val His Met Cys Gln Asp Leu Arg Val Leu Asn Ala Val Arg Asp
        275                 280                 285

Tyr His Ile Gly Ile Pro Leu Thr Tyr Ser Gln Tyr Lys Gln Leu Thr
    290                 295                 300

Ile Gln Val Leu Leu Asp Arg Leu Val Leu Arg Arg Leu Tyr Pro Leu
305                 310                 315                 320

Ala Ile Gln Ile Cys Glu Tyr Leu Arg Leu Pro Glu Val Gln Gly Val
                325                 330                 335

Ser Arg Ile Leu Ala His Trp Ala Cys Tyr Lys Val Gln Gln Lys Asp
            340                 345                 350

Val Ser Asp Glu Asp Val Ala Arg Ala Ile Asn Gln Lys Leu Gly Asp
        355                 360                 365

Thr Pro Gly Val Ser Tyr Ser Asp Ile Ala Ala Arg Ala Tyr Gly Cys
    370                 375                 380

Gly Arg Thr Glu Leu Ala Ile Lys Leu Leu Glu Tyr Glu Pro Arg Ser
385                 390                 395                 400

Gly Glu Gln Val Pro Leu Leu Leu Lys Met Lys Arg Ser Lys Leu Ala
                405                 410                 415

Leu Ser Lys Ala Ile Glu Ser Gly Asp Thr Asp Leu Val Phe Thr Val
            420                 425                 430

Leu Leu His Leu Lys Asn Glu Leu Asn Arg Gly Asp Phe Phe Met Thr
        435                 440                 445

Leu Arg Asn Gln Pro Met Ala Leu Ser Leu Tyr Arg Gln Phe Cys Lys
    450                 455                 460

His Gln Glu Leu Glu Thr Leu Lys Asp Leu Tyr Asn Gln Asp Asp Asn
465                 470                 475                 480

His Gln Glu Leu Gly Ser Phe His Ile Arg Ala Ser Tyr Ala Ala Glu
                485                 490                 495

Glu Arg Ile Glu Gly Arg Val Ala Ala Leu Gln Thr Ala Ala Asp Ala
            500                 505                 510

Phe Tyr Lys Ala Lys Asn Glu Phe Ala Ala Lys Ala Thr Glu Asp Gln
        515                 520                 525

Met Arg Leu Leu Arg Leu Gln Arg Arg Leu Glu Asp Glu Leu Gly Gly
    530                 535                 540

Gln Phe Leu Asp Leu Ser Leu His Asp Thr Val Thr Thr Leu Ile Leu
545                 550                 555                 560

Gly Gly His Asn Lys Arg Ala Glu Gln Leu Ala Arg Asp Phe Arg Ile
                565                 570                 575

Pro Asp Lys Arg Leu Trp Trp Leu Lys Leu Thr Ala Leu Ala Asp Leu
            580                 585                 590

Glu Asp Trp Glu Glu Leu Glu Lys Phe Ser Lys Ser Lys Lys Ser Pro
        595                 600                 605

Ile Gly Tyr Leu Pro Phe Val Glu Ile Cys Met Lys Gln His Asn Lys
    610                 615                 620

Tyr Glu Ala Lys Lys Tyr Ala Ser Arg Val Gly Pro Glu Gln Lys Val
625                 630                 635                 640

Lys Ala Leu Leu Leu Val Gly Asp Val Ala Gln Ala Ala Asp Val Ala
                645                 650                 655

Ile Glu His Arg Asn Glu Ala Glu Leu Ser Leu Val Leu Ser His Cys
            660                 665                 670

Thr Gly Ala Thr Asp Gly Ala Thr Ala Asp Lys Ile Gln Arg Ala Arg
        675                 680                 685

Ala Gln Ala Gln Lys Lys
    690

<210>  23
<211>  492

<212>  PRT
<213>  Homo Sapiens

<400>  23

<210>  24
<211>  439
<212>  PRT
<213>  Homo Sapiens

<400>  24

Thr Pro Leu Pro Pro Thr Ser Ala His Gly Asn Val Ala Glu Gly Glu
1               5                   10                  15

Thr Lys Pro Asp Pro Asp Val Thr Glu Arg Cys Ser Asp Gly Trp Ser
            20                  25                  30

Phe Asp Ala Thr Thr Leu Asp Asp Asn Gly Thr Met Leu Phe Phe Lys
        35                  40                  45

Gly Glu Phe Val Trp Lys Ser His Lys Trp Asp Arg Glu Leu Ile Ser
    50                  55                  60

Glu Arg Trp Lys Asn Phe Pro Ser Pro Val Asp Ala Ala Phe Arg Gln
65                  70                  75                  80

Gly His Asn Ser Val Phe Leu Ile Lys Gly Asp Lys Val Trp Val Tyr
                85                  90                  95

Pro Pro Glu Lys Lys Glu Lys Gly Tyr Pro Lys Leu Leu Gln Asp Glu
            100                 105                 110

Phe Pro Gly Ile Pro Ser Pro Leu Asp Ala Ala Val Glu Cys His Arg
        115                 120                 125

Gly Glu Cys Gln Ala Glu Gly Val Leu Phe Phe Gln Gly Asp Arg Glu
    130                 135                 140

Trp Phe Trp Asp Leu Ala Thr Gly Thr Met Lys Glu Arg Ser Trp Pro
145                 150                 155                 160

Ala Val Gly Asn Cys Ser Ser Ala Leu Arg Trp Leu Gly Arg Tyr Tyr
                165                 170                 175

Cys Phe Gln Gly Asn Gln Phe Leu Arg Phe Asp Pro Val Arg Gly Glu
            180                 185                 190

Val Pro Pro Arg Tyr Pro Arg Asp Val Arg Asp Tyr Phe Met Pro Cys
        195                 200                 205

Pro Gly Arg Gly His Gly His Arg Asn Gly Thr Gly His Gly Asn Ser
    210                 215                 220

Thr His His Gly Pro Glu Tyr Met Arg Cys Ser Pro His Leu Val Leu
225                 230                 235                 240

Ser Ala Leu Thr Ser Asp Asn His Gly Ala Thr Tyr Ala Phe Ser Gly
                245                 250                 255

Thr His Tyr Trp Arg Leu Asp Thr Ser Arg Asp Gly Trp His Ser Trp
            260                 265                 270

Pro Ile Ala His Gln Trp Pro Gln Gly Pro Ser Ala Val Asp Ala Ala
        275                 280                 285

Phe Ser Trp Glu Glu Lys Leu Tyr Leu Val Gln Gly Thr Gln Val Tyr
    290                 295                 300

Val Phe Leu Thr Lys Gly Gly Tyr Thr Leu Val Ser Gly Tyr Pro Lys
305                 310                 315                 320

Arg Leu Glu Lys Glu Val Gly Thr Pro His Gly Ile Ile Leu Asp Ser
                325                 330                 335

Val Asp Ala Ala Phe Ile Cys Pro Gly Ser Ser Arg Leu His Ile Met
            340                 345                 350

Ala Gly Arg Arg Leu Trp Trp Leu Asp Leu Lys Ser Gly Ala Gln Ala
        355                 360                 365

Thr Trp Thr Glu Leu Pro Trp Pro His Glu Lys Val Asp Gly Ala Leu
    370                 375                 380

Cys Met Glu Lys Ser Leu Gly Pro Asn Ser Cys Ser Ala Asn Gly Pro
385                 390                 395                 400

Gly Leu Tyr Leu Ile His Gly Pro Asn Leu Tyr Cys Tyr Ser Asp Val
                405                 410                 415

Glu Lys Leu Asn Ala Ala Lys Ala Leu Pro Gln Pro Gln Asn Val Thr
            420                 425                 430

Ser Leu Leu Gly Cys Thr His
        435

<210>  25
<211>  431
<212>  PRT
<213>  Homo Sapiens

<400>  25

Lys Met Ala Ala Pro Met Glu Val Ala Val Cys Thr Asp Ser Ala Ala
1               5                   10                  15

Pro Met Trp Ser Cys Ile Val Trp Glu Leu His Ser Gly Ala Asn Leu
            20                  25                  30

Leu Thr Tyr Arg Gly Gly Gln Ala Gly Pro Arg Gly Leu Ala Leu Leu
        35                  40                  45

Asn Gly Glu Tyr Leu Leu Ala Ala Gln Leu Gly Lys Asn Tyr Ile Ser
    50                  55                  60

Ala Trp Glu Leu Gln Arg Lys Asp Gln Leu Gln Gln Lys Ile Met Cys

65                  70                  75                  80

Pro Gly Pro Val Thr Cys Leu Thr Ala Ser Pro Asn Gly Leu Tyr Val
                85                  90                  95

Leu Ala Gly Val Ala Glu Ser Ile His Leu Trp Glu Val Ser Thr Gly
            100                 105                 110

Asn Leu Leu Val Ile Leu Ser Arg His Tyr Gln Asp Val Ser Cys Leu
        115                 120                 125

Gln Phe Thr Gly Asp Ser Ser His Phe Ile Ser Gly Gly Lys Asp Cys
    130                 135                 140

Leu Val Leu Val Trp Ser Leu Cys Ser Val Leu Gln Ala Asp Pro Ser
145                 150                 155                 160

Arg Ile Pro Ala Pro Arg His Val Trp Ser His His Ala Leu Pro Ile
                165                 170                 175

Thr Asp Leu His Cys Gly Phe Gly Gly Pro Leu Ala Arg Val Ala Thr
            180                 185                 190

Ser Ser Leu Asp Gln Thr Leu Trp Glu Val Ser Ser Gly Glu Leu Leu
        195                 200                 205

Leu Ser Val Leu Phe Asp Val Ser Ile Met Ala Val Thr Met Asp Leu
    210                 215                 220

Ala Glu His His Met Phe Cys Gly Gly Ser Glu Gly Ser Ile Phe Gln
225                 230                 235                 240

Val Asp Leu Phe Thr Trp Pro Gly Gln Arg Glu Arg Ser Phe His Pro
                245                 250                 255

Glu Gln Asp Ala Gly Lys Val Phe Lys Gly His Arg Asn Gln Val Thr
            260                 265                 270

Cys Leu Ser Val Ser Thr Asp Gly Ser Val Leu Leu Ser Gly Ser His
        275                 280                 285

Asp Glu Thr Val Arg Leu Trp Asp Val Gln Ser Lys Gln Cys Ile Arg
    290                 295                 300

Thr Val Ala Leu Lys Gly Pro Val Thr Asn Ala Ala Ile Leu Leu Ala
305                 310                 315                 320

Pro Val Ser Met Leu Ser Ser Asp Phe Arg Pro Ser Leu Pro Leu Pro
                325                 330                 335

His Phe Asn Lys His Leu Leu Gly Ala Glu His Gly Asp Glu Pro Arg

340                 345                 350

His Gly Gly Leu Thr Leu Arg Leu Gly Leu His Gln Gln Gly Ser Glu
        355                 360                 365

Pro Ser Tyr Leu Asp Arg Thr Glu Gln Leu Gln Ala Val Leu Cys Ser
    370                 375                 380

Thr Met Glu Lys Ser Val Leu Gly Gly Gln Asp Gln Leu Arg Val Arg
385                 390                 395                 400

Val Thr Glu Leu Glu Asp Glu Val Arg Asn Leu Arg Lys Ile Asn Arg
                405                 410                 415

Asp Leu Phe Asp Phe Ser Thr Arg Phe Ile Thr Arg Pro Ala Lys
            420                 425                 430

<210>  26
<211>  601
<212>  PRT
<213>  Homo Sapiens

<400>  26

Pro Ser Gly Lys Met Ala Ala Val Gln Ala Ala Glu Val Lys Val Asp
1               5                   10                  15

Gly Ser Glu Pro Lys Leu Ser Lys Asn Glu Leu Lys Arg Arg Leu Lys
            20                  25                  30

Ala Glu Lys Lys Val Ala Glu Lys Glu Ala Lys Gln Lys Glu Leu Ser
        35                  40                  45

Glu Lys Gln Leu Ser Gln Ala Thr Ala Ala Ala Thr Asn His Thr Thr
    50                  55                  60

Asp Asn Gly Val Gly Pro Glu Glu Glu Ser Val Asp Pro Asn Gln Tyr
65                  70                  75                  80

Tyr Lys Ile Arg Ser Gln Ala Ile His Gln Leu Lys Val Asn Gly Glu
                85                  90                  95

Asp Pro Tyr Pro His Lys Phe His Val Asp Ile Ser Leu Thr Asp Phe
            100                 105                 110

Ile Gln Lys Tyr Ser His Leu Gln Pro Gly Asp His Leu Thr Asp Ile
        115                 120                 125

Thr Leu Lys Val Ala Gly Arg Ile His Ala Lys Arg Ala Ser Gly Gly
    130                 135                 140

Lys Leu Ile Phe Tyr Asp Leu Arg Gly Glu Gly Val Lys Leu Gln Val
145                 150                 155                 160

Met Ala Asn Ser Arg Asn Tyr Lys Ser Glu Glu Glu Phe Ile His Ile
                165                 170                 175

Asn Asn Lys Leu Arg Arg Gly Asp Ile Ile Gly Val Gln Gly Asn Pro
            180                 185                 190

Gly Lys Thr Lys Lys Gly Glu Leu Ser Ile Ile Pro Tyr Glu Ile Thr
        195                 200                 205

Leu Leu Ser Pro Cys Leu His Met Leu Pro His Leu His Phe Gly Leu
    210                 215                 220

Lys Asp Lys Glu Thr Arg Tyr Arg Gln Arg Tyr Leu Asp Leu Ile Leu
225                 230                 235                 240

Asn Asp Phe Val Arg Gln Lys Phe Ile Ile Arg Ser Lys Ile Ile Thr
                245                 250                 255

Tyr Ile Arg Ser Phe Leu Asp Glu Leu Gly Phe Leu Glu Ile Glu Thr
            260                 265                 270

Pro Met Met Asn Ile Ile Pro Gly Gly Ala Val Ala Lys Pro Phe Ile
        275                 280                 285

Thr Tyr His Asn Glu Leu Asp Met Asn Leu Tyr Met Arg Ile Ala Pro
    290                 295                 300

Glu Leu Tyr His Lys Met Leu Val Val Gly Gly Ile Asp Arg Val Tyr
305                 310                 315                 320

Glu Ile Gly Arg Gln Phe Arg Asn Glu Gly Ile Asp Leu Thr His Asn
                325                 330                 335

Pro Glu Phe Thr Thr Cys Glu Phe Tyr Met Ala Tyr Ala Asp Tyr His
            340                 345                 350

Asp Leu Met Glu Ile Thr Glu Lys Met Val Ser Gly Met Val Lys His
        355                 360                 365

Ile Thr Gly Ser Tyr Lys Val Thr Tyr His Pro Asp Gly Pro Glu Gly
    370                 375                 380

Gln Ala Tyr Asp Val Asp Phe Thr Pro Pro Phe Arg Arg Ile Asn Met
385                 390                 395                 400

Val Glu Glu Leu Glu Lys Ala Leu Gly Met Lys Leu Pro Glu Thr Asn
                405                 410                 415

Leu Phe Glu Thr Glu Glu Thr Arg Lys Ile Leu Asp Asp Ile Cys Val
            420                 425                 430

Ala Lys Ala Val Glu Cys Pro Pro Pro Arg Thr Thr Ala Arg Leu Leu
        435                 440                 445

Asp Lys Leu Val Gly Glu Phe Leu Glu Val Thr Cys Ile Asn Pro Thr
    450                 455                 460

Phe Ile Cys Asp His Pro Gln Ile Met Ser Pro Leu Ala Lys Trp His
465                 470                 475                 480

Arg Ser Lys Glu Gly Leu Thr Glu Arg Phe Glu Leu Phe Val Met Lys
                485                 490                 495

Lys Glu Ile Cys Asn Ala Tyr Thr Glu Leu Asn Asp Pro Met Arg Gln
            500                 505                 510

Arg Gln Leu Phe Glu Glu Gln Ala Lys Ala Lys Ala Ala Gly Asp Asp
        515                 520                 525

Glu Ala Met Phe Ile Asp Glu Asn Phe Cys Thr Ala Leu Glu Tyr Gly
    530                 535                 540

Leu Pro Pro Thr Ala Gly Trp Gly Met Gly Ile Asp Arg Val Ala Met
545                 550                 555                 560

Phe Leu Thr Asp Ser Asn Asn Ile Lys Glu Val Leu Leu Phe Pro Ala
                565                 570                 575

Met Lys Pro Glu Asp Lys Lys Glu Asn Val Ala Thr Thr Asp Thr Leu
            580                 585                 590

Glu Ser Thr Thr Val Gly Ser Ser Val
        595                 600

## Beschreibung

Die vorliegende Erfindung betrifft neue Markersequenzen für Adipositas (synonym: Obesitas, engl. obesity (auch: Fettleibigkeit, starkes Übergewicht, Fettsucht)) und deren diagnostische Verwendung samt einem Verfahren zum Screenen von potentiellen Wirkstoffen für Adipositas mittels dieser Markersequenzen. Ferner betrifft die Erfindung eine diagnostische Vorrichtung enthaltend solche Markersequenzen für Adipositas, insbesondere ein Proteinbiochip und dessen Verwendung.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Proteinbiochips weisen vorteilhaft eine hohe Empfindlichkeit aus.

Es besteht jedoch ein hohes Bedürfnis indikationsspezifische diagnostische Vorrichtungen, wie einen Proteinbiochip, bereitzustellen.

Die Aufgabe der vorliegenden Erfindung ist die Bereitstellung von verbesserten Markersequenzen und deren diagnostische Verwendung zur Behandlung von Adipositas.

Die Aufgabe wird erfindungsgemäß gelöst durch die Bereitstellung von SEQ 1 - 26 neuen Markersequenzen, die erstmalig mittels eines Proteinbiochips, insbesondere samt bioinformatorischer Auswertung ermittelt werden konnte. Daher wurden die SEQ 1 - 26 mit Hilfe eines Proteinbiochips erstmalig identifiziert.

Die Bereitstellung von spezifischen Markersequenzen erlaubt eine sichere Diagnose und Stratifizierung von Patienten mit Adipositas, insbesondere mittels eines Proteinbiochips.

Daher betrifft die Erfindung die Verwendung von Markersequenzen zur Diagnose von Adipositas, wobei mindestens eine Markersequenz eines Peptids ausgewählt aus der Gruppe SEQ 1 - 26 oder jeweils eine dafür kodierende cDNA oder jeweils einer Teilsequenz oder Fragment davon (nachstehend: erfindungsgemäße Markersequenzen) an oder von einem zu untersuchenden Patienten bestimmt wird.

Die erfindungsgemäßen Markersequenzen konnten mittels differentiellem Screenen von Proben und zwar gesunder Probanden mit Patientenproben mit Adipositas identifiziert werden.

Der Begriff "Adipositas" (synonym: Obesitas, Fettleibigkeit, starkes Übergewicht, Fettsucht) bedeutet krankhaftes Übergewicht, das zu einer gesundheitlichen Beeinträchtigung führt und ein Risikofaktor für Folgeerkrankungen, wie das metabolische Syndrom samt Diabetes mellitus, Hyperlipidämie, Hypertonie, Arteriosklerose und Gicht darstellt. Symptome: erhöhter Körperfettanteil (normal ca. 15-18 % beim Mann, 20-25 % bei der Frau), näherungsweise bestimmt mit Body*-mass-Index (Definition z.B. nach Pschyrembel, de Gruyter, 261. Auflage (2007), Berlin).

In einer weiteren Ausführungsform werden daher mindestens 2 bis 5 oder 10, vorzugsweise 20 Markersequenzen oder mehr Markersequenzen an oder von einem zu untersuchenden Patienten bestimmt.

In einer weiteren Ausführungsform der Erfindung können die erfindungsgemäßen Markersequenzen ebenfalls mit bekannten Biomarkern für diese Indikation kombiniert, ergänzt oder erweitert werden.

In einer bevorzugten Ausführungsform erfolgt die Bestimmung der Markersequenzen außerhalb des menschlichen Körpers und die Bestimmung erfolgt in einer ex vivo / in vitro Diagnose.

In einer weiteren Ausführungsform der Erfindung betrifft die Erfindung die Verwendung von Markersequenzen als Diagnostika, wobei mindestens eine Markersequenz eines Peptids ausgewählt aus der Gruppe SEQ 1 - 26 oder jeweils eine dafür kodierende cDNA oder jeweils einer Teilsequenz oder Fragment davon ist.

Ferner betrifft die Erfindung ein Verfahren zur Diagnose von Adipositas, wobei a.) mindestens eine Markersequenz eines Peptids ausgewählt aus der Gruppe SEQ 1 - 26 oder jeweils eine dafür kodierende cDNA oder jeweils einer Teilsequenz oder Fragment davon auf einem festen Träger aufgebracht wird und b.) mit Körperflüssigkeit oder Gewebeauszug eines Patienten in Kontakt gebracht wird und c.) der Nachweis einer Wechselwirkung der Körperflüssigkeit oder Gewebeauszug mit den Markersequenzen aus a.) erfolgt.

Daher betrifft die Erfindung ebenfalls Diagnostika zur Diagnose von Adipositas jeweils ausgewählt aus der Gruppe SEQ 1 - 26 oder jeweils ein dafür kodierendes Protein oder jeweils einer Teilsequenz oder Fragment davon.

Der Nachweis einer solchen Wechselwirkung kann beispielsweise durch eine Sonde, insbesondere durch einen Antikörper erfolgen.

Daher betrifft die Erfindung ebenfalls die Aufgabe eine diagnostische Vorrichtung oder einen Assay, insbesondere einen Proteinbiochip, bereitzustellen, der für Adipositas eine Diagnose oder Untersuchung erlaubt.

Ferner betrifft die Erfindung ein Verfahren zum Stratifizieren, insbesondere zur Risikostratifizierung und / oder Therapiesteuerung eines Patienten mit Adipositas, wobei mindestens eine Markersequenz eines Peptids ausgewählt aus der Gruppe SEQ 1 - 26 oder jeweils eine dafür kodierende cDNA an oder von einem zu untersuchenden Patienten bestimmt wird.

Ferner umfasst ist die Stratifizierung der Patienten mit Adipositas in neue oder etablierte Subgruppen der Adipositas, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

"Diagnose" im Sinne dieser Erfindung bedeutet die positive Feststellung der Adipositas mittels der erfindungsgemäßen Markersequenzen sowie die Zuordnung der Patienten zu Adipositas. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose ebenfalls die Differentialdiagnose von Adipositas mittels der erfindungsgemäßen Markersequenzen sowie die Prognose von Adipositas.

"Stratifizieren (auch: Stratifikation) oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Adipositas untersucht wird.

Der Begriff "Markersequenzen" im Sinne dieser Erfindung bedeutet, dass die cDNA oder das jeweilige Polypeptid oder Protein signifikant für Adipositas sind. Beispielsweise können die cDNA oder das jeweilige Polypeptid oder Protein eine Wechselwirkung mit Substanzen aus der Körperflüssigkeit oder Gewebeauszug eines Patienten mit Adipositas aufweisen (z.B. Antigen (Epitop) / (Auto)Antikörper (Paratop) Wechselwirkung). Im Sinne der Erfindung bedeutet "wobei mindestens eine Markersequenz eines Peptids ausgewählt aus der Gruppe SEQ 1 - 26 oder jeweils eine dafür kodierende cDNA oder jeweils einer Teilsequenz oder Fragment davon an oder von einem zu untersuchenden Patienten bestimmt wird", dass eine Wechselwirkung zwischen der Körperflüssigkeit oder Gewebeauszuges eines Patienten und den erfindungsgemäßen Markersequenzen nachgewiesen wird. Eine solche Wechselwirkung ist z.B. eine Bindung, insbesondere eine bindende Substanz an mindestens einer erfindungsgemäßen Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

In einer weiteren Ausführungsform der Erfindung können jedoch die erfindungsgemäßen Markersequenzen in einer signifikant höheren oder niedrigeren Expressionsrate oder Konzentration vorliegen, dass auf Adipositas hinweist. Hierbei wird mittels Proteomics oder Nukleinsäureblots die relativen Expressionsraten krank / gesund der erfindungsgemäßen Markersequenzen für Adipositas bestimmt.

Die Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen sind Gegenstand der Tabelle A und können durch den jeweilig zitierten Datenbankeintrag (auch mittels Internet: http://www.ncbi.nlm.nih.gov/) eindeutig identifiziert werden (siehe in Tabelle A: dort Accession No.), siehe ebenfalls das zugehörige Sequenzprotokoll.

Daher betrifft die Erfindung ebenfalls die Volllängesequenzen der erfindungsgemäßen Marker und zwar wie in Tabelle A über den bekannten Datenbankeintrag definiert, nachstehend SEQ 1a-26a genannt.

Weiterhin umfasst sind daher ebenfalls analoge Ausführungsformen von SEQ 1a-26a zu den Markersequenzen SEQ 1-26, wie z.B. in den Ansprüchen dargelegt, da die erfindungsgemäßen SEQ 1-26 wiederum Teilsequenzen, zumindest mit hoher Homologie, darstellen. Die spezifischen Markersequenzen SEQ 1-26 sind jedoch erfindungsgemäß bevorzugt.

Erfindungsgemäß umfassen die Markersequenzen auch solche Modifikationen der cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

In einer weiteren Ausführungsform der Erfindung sind ebenfalls Teilsequenzen oder Fragmente der erfindungsgemäßen Markersequenzen umfasst. Insbesondere solche Teilsequenzen, die eine Identität von 95%, 90 %, insbesondere 80% oder 70 % mit den erfindungsgemäßen Markersequenzen aufweisen.

Teilsequenzen sind ebenfalls solche Sequenzen, die 50 bis 100 Aminosäuren, 70-120 Aminosäuren einer Sequenz der SEQ 1-26 aufweisen, oder davon erhältliche Peptide.

In einer weiteren Ausführungsform kann die jeweilige Markersequenz in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Ein weiterer Gegenstand der Erfindung betrifft eine Anordnung von Markersequenzen enthaltend mindestens eine Markersequenz eines Peptids ausgewählt aus der Gruppe SEQ 1 - 26 oder jeweils ein dafür kodierendes Protein. Vorzugsweise enthält die Anordnung mindestens 2 bis 5 oder 10, vorzugsweise 20 oder mehr Markersequenzen.

Im Rahmen dieser Erfindung bedeutet "Anordnung" synonym "Array" und sofern dieser "Array" zur Identifizierung von Substanzen an Markersequenzen verwendet wird, ist hierunter ein "Assay" oder eine diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Proteinbindern erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren. Ein Proteinbiochip im Sinne dieser Erfindung ist die systematische Anordnung von Proteinen auf einem festen Träger.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip bestehend aus einer Anordnung enthaltend erfindungsgemäße Markersequenzen.

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60 (5) :523-33) .

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden.

Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einen festen Träger fixiert, gespottet oder immobilisiert.

Daher betrifft die Erfindung eine Anordnung, wobei die Markersequenzen als Clone vorliegen.

Zusätzlich können die Markersequenzen in der jeweiligen Form in Form eines Fusionsproteins vorliegen, welches beispielsweise mindestens ein Affinitätsepiptop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz für Adipositas, **dadurch gekennzeichnet, dass** eine erfindungsgemäße Anordnung oder Assay mit a.) mindestens einer zu untersuchenden Substanz in Kontakt gebracht wird und b.) ein Bindungserfolg nachgewiesen wird.

Ferner betrifft die Erfindung ein Verfahren zum Identifizieren und Charakterisieren einer Substanz für Adipositas, **dadurch gekennzeichnet, dass** eine erfindungsgemäße Anordnung oder Assay mit a.) mindestens einer zu untersuchenden Substanz in Kontakt gebracht wird und b.) ein Bindungserfolg nachgewiesen wird.

Die zu untersuchende Substanz kann ein beliebiges natives oder nicht-natives Biomolekül, ein synthetisches chemisches Molekül, eine Mischung oder eine Substanzbibliothek sein.

Nachdem die zu untersuchende Substanz eine Markersequenz kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Adipositas entwickelt und erhältlich durch den Einsatz des erfindungsgemäßen Assays oder Proteinbiochip.

Daher betrifft die Erfindung ebenfalls die Verwendung einer erfindungsgemäßen Anordnung oder einem Assay zum Screenen von Wirkstoffen für Adipositas.

Daher betrifft die Erfindung in einer weiteren Ausführungsform ebenfalls ein Target zur Behandlung und Therapie von Adipositas, jeweils ausgewählt aus der Gruppe SEQ 1 - 26 oder jeweils eine dafür kodierende cDNA.

In einer weiteren Ausführungsform betrifft die Erfindung ebenfalls die Verwendung der erfindungsgemäßen Markersequenzen, vorzugsweise in Form einer Anordnung, als Affinitätsmaterial zur Durchführung einer Apherese bzw. iwS. einer Blutwäsche, wobei Substanzen aus Körperflüssigkeiten eines Patienten mit Adipositas, wie Blut oder Plasma, an die erfindungsgemäßen Markersequenzen binden und folglich der Körperflüssigkeit selektiv entzogen werden können.

### Beispiele und Figuren:

Zehn oder mehr Patientenproben wurden individuell gegen eine cDNA Expressionsbibliothek gescreent. Die Adipositas - spezifischen Expressionsklone wurden ermittelt durch einen Vergleich mit zehn oder mehr gesunden Proben. Die Identität der Markersequenzen wurde durch DNA-Sequenzierung ermittelt.

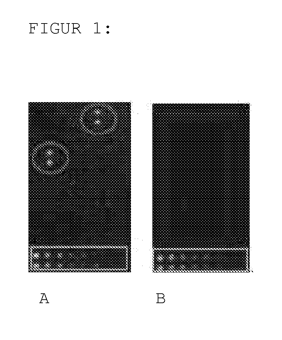

In Figur 1 wird das differentielle Screenen zwischen zwei Proteinbiochips aus jeweils einer cDNA-Expressionsbank eines Patienten und einem gesunden Probanden gezeigt. Die differentiellen Clone werden mittels Fluoresenzmarkierung nachgewiesen und bioinformatorisch ausgewertet.

Im Rahmen der Biomarkeridentifizierung werden verschiedene bioinformatische Analysen durchgeführt. Für jedes Serum werden mittels Microarray Reaktivitäten gegen ca. 2000 unterschiedliche Antigene gemessen. Diese Daten werden für ein Ranking der gespotteten Antigene bzgl. ihrer Differenzierungsfähigkeit zwischen gesunden und erkrankten Seren benutzt. Diese Auswertung wird mittels des nicht parametrischen Mann-Whitney Tests auf normalisierten Intensitätsdaten durchgeführt. Zur Normalisierung wird ein interner Standard benutzt, der auf jedem Chip mitgespottet wird. Da für jedes Antigen ein p-Wert berechnet wird, werden Methoden zur Korrektur des multiples Testens eingesetzt. Als sehr konservativer Ansatz wird eine Bonferroni Korrektur durchgeführt und zusätzlich wird die weniger restriktive False Discovery Rate (FDR) nach Benjamini & Hochberg berechnet.

Desweiteren werden die Daten zur Klassifikation der Seren benutzt. Hierbei kommen unterschiedliche multivariate Methoden zum Einsatz. Dies sind Methoden aus den statistischen Lernverfahren wie Support Vector Machines (SVM), Neuronale Netze oder Klassifikationsbäume, sowie eine

Schwellenwertmethode, welche sowohl zur Klassifikation als auch zur visuellen Repräsentation der Daten geeignet ist.

Zur Vermeidung von Overfitting wird eine 10fache Cross-Validierung der Daten durchgeführt.

### Zum Vorgehen:

Autoantikörper sind pathognomisch für Autoimmunerkrankungen, treten aber auch bei Erkrankungen auf, die nicht im klassischen Sinne zu den Autoimmunerkrankungen zählen. Auch Gesunde verfügen über viele Autoantikörper, die nicht mit einer erkennbaren Krankheitsaktivität assoziiert sind.

An der Entstehung von Antikörpern sind mehrere Mechanismen beteiligt. So gelangt z.B. phagozytiertes Fremdmaterial (Krankheitserreger etc), über den endosomalen lysomalen Pathway in das Lysosom, in dem es durch intrazelluläre Verdauungsenzyme degradiert wird. Proteine werden dabei zu Peptiden gespalten. Einige der entstandenen Peptide gelangen anschließend in MHC Klasse II Kompartimente und werden von den MHC Klasse II-Rezeptoren gebunden und an die Plasmamembran transportiert. Dort werden sie dem Immunsystem präsentiert, so dass Antikörper gegen die Peptide gebildet werden.

Neben Fremdantigenen werden aber auch zelleigene Antigene dem Immunsystem präsentiert mit folgender Bildung von Autoantikörpern. Ein Mechanismus, der an der Präsentation intrazellulärer Antigene beteiligt ist, ist die Autophagie. Im Rahmen der Makroautophagie wird intrazelluläres Material von doppelseitigen Membranen umschlossen. Das entstandene Autophagosom fusioniert mit dem späten Endosom bzw. Lysosom, so dass das Material im Inneren des Vesikels verdaut werden kann. Im Rahmen der Chaperon-vermittelten Autophagie werden bestimmte intrazelluläre Proteine von Chaperonen erkannt, entfaltet und mit Hilfe des lysosomalen Membranproteins LAMP2 in das Lysosom importiert. Beide Mechanismen erzeugen Peptide, die ebenso wie Peptide aus dem Verdau von Fremdmaterial in die MHC Klasse II Kompartimente gelangen, von den MHC Klasse II-Rezeptoren gebunden und an der Plasmamembran dem Immunsystem präsentiert werden.

Hungernde Zellen decken ihren Energiebedarf u.a. durch Steigerung der Autophagie und dem damit verbundenen Verdau intrazellulärer Proteine und Organellen ab. Innerhalb der ersten 24h wird insbesondere die (unselektive) Makroautophagie gesteigert, während nach längerem Fasten die selektive Chaperon-vermittelte Autophagie in den Vordergrund tritt. Im Rahmen des Hungerns verändern sich auch die Peptide die von MHC Klasse II-Rezeptoren an der Plasmamembran präsentiert werden.

Erfindungsgemäß wurde die Veränderung der Antikörperprofile in adipösen Patienten während einer deutlichen Gewichtsabnahme bestimmt. Dazu wurden wir mit dem Unichip® AV-400 (Protagen AG, Dortmund) Seren von Adipösen, die an einem Gewichtsreduktionsprogramm teilnehmen, vor und nach Gewichtsverlust untersucht. Als Kontrollen dienten normalgewichtige Individuen ohne Gewichtsveränderungen.

### Rekrutierung der Adipösen und der normalgewichtigen Kontrollen:

In die Studie eingeschlossen wurden adipöse Individuen, die an einem organisierten Gewichtsreduktionsprogramm teilnahmen. Einschlusskriterien waren ein BMI > 30 kg/m² und ein Gewichtsverlust von > 10% innerhalb der ersten drei Monate. Um Veränderungen in den Antikörperprofilen zu dokumentieren, wurde den Studienteilnehmern vor und nach Gewichtsverlust Blut in einer Serummonovette abgenommen. Als Kontrollen dienten normalgewichtige (BMI < 25 kg/m²), gesunde Individuen ohne Gewichtsveränderungen (< ± 1kg), denen im Abstand von drei Monaten ebenfalls Blut in einer Serummonovette entnommen wurde.

### Unichip® AV-400 (Proteinbiochip):

Der Unichip® AV-400 ist ein planarer Proteinarray und enthält 400 unterschiedliche humane Proteine sowie zusätzliche Proteine zur Kontrolle des Assays. Die Proteine bilden einen Querschnitt aller Zellproteine und stammen aus unterschiedlichen pharmazeutisch wichtigen Proteinklassen wie etwa Kinasen, Membran-, Signal- und Stoffwechselproteine. Ausgewählt wurden sie mittels der Datenbank Gene Ontology, die u.a. zelluläre Proteine einzelnen zellulären Funktionen zuordnet. Aus jedem Bereich wurden so viele Proteine zufällig ausgewählt, dass ihre Gesamtzusammensetzung ein repräsentatives Abbild der dort beschriebenen Aufteilung darstellt.

### Chipherstellung und Assay-Schema:

Die Proteine werden auf einen modifizierten Objektträger gedruckt. Der Nachweis möglicher humaner Antikörper gegen die Antigene auf dem Protein-Biochip erfolgt in zwei Stufen. In einem ersten Inkubationsschritt wird der Protein-Biochip mit dem menschlichen Serum inkubiert, so dass vorhandene menschliche Antikörper die Proteine auf dem Biochip binden. In einem zweiten Inkubationsschritt werden gebundene humane Antikörper der Klasse IgG von Maus-IgG-Antikörpern erkannt. Die Maus-IgG-Antikörper sind wiederum in einer Vorreaktion mit Anti-IgG-Maus-Antikörpern gekoppelt worden, an denen sich ein fluoreszierender Farbstoff befindet.
Durch Detektion des Fluoreszenzsignals lassen sich dann die gebundenen IgG-Antikörper aus dem menschlichen Serum nachweisen.

### Identifizierung neuer Antigene

Bei jedem Studienteilnehmer (Adipöse und Kontrollen) wurde im Abstand von drei Monaten zweimal Blut in einer Serummonovette entnommen. Die Seren wurden mit dem Unichip® AV-400 inkubiert, so dass für jeden Studienteilnehmer pro Antigen auf dem Chip zwei Antikörpertiter bestimmt wurden, die im Folgenden als Relative Units (RU) bezeichnet werden. Um nun die Veränderung der Antikörpertiter zu erfassen, wurde für jeden Patienten und jedes Antigen der Quotient aus dem Antikörpertitern vor und nach den drei Monaten gebildet. Dieser Quotient wurde dann noch zur Basis 2 logarithmiert. Negative Ergebnisse dokumentieren einen Abfall, positive Ergebnisse einen Anstieg der Antikörpertiter.

Zur Identifikation von Antigenen, gegen die transiente Antikörper gebildet wurden, wurden verschiedene statistische Tests angewendet. Die Ergebnisse der Adipösen und der Kontrollen wurden sowohl mit dem T-Test als auch Wilcox-Mann-Withney-Test auf signifikante Unterschiede untersucht. Die Analyse erfolgte in beiden Fällen einseitig, da davon ausgegangen wurde, dass es bei den Adipösen im Rahmen der Gewichtsabnhame und Autophagieaktivierung zu einer vermehrten Antikörperbildung gegen die Zielantigene kommt. Die Irrtumswahrscheinlichkeit wurde in beiden Fällen auf 5% gesetzt. Zum Ausschlus einer Alpha-Fehler-Kumulierung wurde die Analyse einer Bonferroni-Korrektur unterzogen. Im Rahmen einer dritte Auswertungsmethode, die unter identifizierte Antigene als "MW + 2* SD" bezeichntet wird, wurden von den Kontrollergebnissen (logarithmierte Quotienten) Mittelwerte und Standardabweichungen berechnet. Als relevant wurden nun die Antigene interpretiert, bei denen mindestens 40 % der Adipösen Ergebnisse (logarithmierte Quotienten) oberhalb der 2x-fachen Standardabweichung aufwiesen. Um zu dokumentieren, dass die tatsächlich vorliegende Verteilung der Kontrollen auf die Kontrollgruppe und der Adipösen auf die Adipösengruppe, die Verteilung ist, bei der die meisten Antigene identifiziert werden, wurde die Analyse nach Randomisierung der Studienteilnehmer wiederholt. Adipöse und Kontrollen wurden vermischt und ohne Zurücklegen zufällig auf die beiden Gruppen neu verteilt. In insgesamt 10000 Randomisierungen wurde lediglich in 5 Fällen mehr Antigene identifiziert als in der nicht randomisierten Analyse. In 9990 Fällen wurden überhaupt keine Antigene identifiziert.

Die identifizierten Antigene sind die erfindungsgemäßen Markersequenzen.

**Tabelle A:**

| SEQ | Acc. No. | **EMBL** | **Funktion** |
|---|---|---|---|
| 1a | gil\|7148545 | ENSG00000011198 | abhydrolase domain containing 5, Lipidkatabolismus |
| 2a | gi\|21328446 | ENSG00000079435 | lipase, hormone-sensitive, Lipidkatabolismus |
| 3a | gi\|13477281 | ENSG00000041988 | THAP domain containing, apoptosis associated protein 3 |
| 4a | gi\|22538467 | ENSG00000159377 | proteasome (prosome, macropain) subunit, beta type, 4 |
| 5a | gi\|1170519 | - | Proteasome activator complex subunit 1 |
| 6a | gi\|21740096 | ENSG00000168374 | ADP-ribosylation factor 4 |
| 7a | gi\|5803102 | ENSG00000185787 | mortality factor 4 like 1 |
| 8a | gi\|13128968 | ENSG00000133878 | dual specificity phosphatase 26 (putative) |
| 9a | gi\|7656926 | ENSG00000054116 | trafficking protein particle complex 3 |
| 10a | gi\|21410307 | ENSG00000168374 | synaptotagmin III, Vesikulärer Transport |
| 11a | gi\|23271403 | ENSG00000105223 | phospholipase D family, member 3 |
| 12a | gi\|39644618 | ENSG00000184014 | DENN/MADD domain containing 5A |
| 13a | gi\|14210504 | ENSG00000072958 | adaptor-related protein complex 1, mu 1 subunit |
| 14a | gi\|4758756 | ENSG00000187109 | assembly protein 1-like 1 |
| 15a | gi\|34782863 | ENSG00000137200 | FtsJ methyltransferase domain containing 2 |
| 16a | gi\|30582193 | - | eukaryotic translation elongation factor 1 gamma |
| 17a | gi\|14424755 | ENSG00000031698 | seryl-tRNA synthetase |
| 18a | gi\|4503987 | ENSG00000137563 | gamma-glutamyl hydrolase (conjugase, folylpolygammaglutamyl hydrolase) |
| 19a | gi\|49259212 | - | Chain D, Human B Lactate Dehydrogenase Complexed With Nad+ And 4-Hydroxy-1,2,5-Oxadiazole-3-Carboxylic Acid |
| 20a | gi\|4507709 | ENSG00000104522 | tissue specific transplantation antigen P35B, Glycokonjugatkopplung |
| 21a | gi\|21361515 | ENSG00000109790 | kelch-like 5 (Drosophila), Lymphozytenaktivierung |
| 22a | gi\|21740096 | - | - |
| 23a | gi\|21749754 | - | - |
| 24a | gi\|1335098 | - | - |
| 25a | gi\|3025445 | - | - |
| 26a | gi\|505108 | - | - |

## Patentansprüche

1. Verwendung der Markersequenzen zur Diagnose von Adipositas, wobei mindestens eine Markersequenz eines Peptids ausgewählt aus der Gruppe SEQ 1 - 26 und / oder SEQ 1a - 26a oder jeweils eine kodierende cDNA oder jeweils einer Teilsequenz oder Fragment davon an oder von einem zu untersuchenden Patienten bestimmt wird.

2. Verwendung der Markersequenzen zur Diagnose von Adipositas nach Anspruch 1, **dadurch gekennzeichnet**, mindestens 2 bis 5 oder 10, vorzugsweise 20 oder mehr Markersequenzen an oder von einem zu untersuchenden Patienten bestimmt wird.

3. Verwendung der Markersequenzen zur Diagnose von Adipositas nach Anspruch 1 oder 2, **dadurch gekennzeichnet, dass** die Bestimmung mittels in-vitro Diagnose erfolgt.

4. Verwendung einer Markersequenz eines Peptids jeweils ausgewählt aus der Gruppe SEQ 1 - 26 und / oder SEQ 1a - 26a oder jeweils eine dafür kodierende cDNA oder jeweils einer Teilsequenz oder Fragment davon als Diagnostikum.

5. Verwendung der Markersequenzen zur Diagnose von Adipositas nach einem der vorhergehenden Ansprüche, **dadurch gekennzeichnet, dass** die Markersequenzen auf einem festen Träger aufgebracht werden, insbesondere einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix.

6. Verfahren zur Diagnose von Adipositas, wobei
a.) mindestens eine Markersequenz eines Peptids ausgewählt aus der Gruppe SEQ 1 - 26 und / oder SEQ 1a - 26a oder jeweils eine dafür kodierende cDNA oder jeweils einer Teilsequenz oder Fragment davon auf einem festen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

7. Verfahren zum Stratifizieren, insbesondere zur Risikostratifizierung, oder zur Therapiesteuerung eines Patienten mit Adipositas, wobei mindestens eine Markersequenz eines Peptids ausgewählt aus der Gruppe SEQ 1 - 26 und / oder SEQ 1a - 26a oder jeweils eine dafür kodierende cDNA oder jeweils einer Teilsequenz oder Fragment davon an einem zu untersuchenden Patienten bestimmt wird.

8. Verfahren nach Anspruch 7, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

9. Anordnung von Markersequenzen enthaltend mindestens eine Markersequenz eines Peptids ausgewählt aus der Gruppe SEQ 1 - 26 und / oder SEQ 1a - 26a oder jeweils eine dafür kodierende cDNA.

10. Anordnung nach Anspruch 9, **dadurch gekennzeichnet, dass** mindestens 2 bis 5 oder 10, vorzugsweise 20 oder mehr Markersequenzen enthalten sind.

11. Anordnung nach Anspruch 9, **dadurch gekennzeichnet, dass** die Markersequenzen als Clone vorliegen.

12. Assay, Proteinbiochip bestehend aus einer Anordnung nach Anspruch 9, **dadurch gekennzeichnet, dass** die Markersequenzen auf einem festen Träger aufgebracht sind.

13. Verwendung einer Anordnung nach einem der Ansprüche 9 bis 11 oder einem Assay nach Anspruch 12 zum Identifizieren und Charakterisieren einer Substanz für Adipositas enthaltend Mittel zum Nachweis eines Bindungserfolges, **dadurch gekennzeichnet, dass** eine Anordnung oder Assay mit a.) mindestens einer zu untersuchenden Substanz in Kontakt gebracht wird und b.) ein Bindungserfolg nachgewiesen wird.

14. Verwendung einer Anordnung nach einem der Ansprüche 9 bis 11 oder einem Assay nach Anspruch 12 zum Screenen von Wirkstoffen für Adipositas.

15. Diagnostika zur Diagnose von Adipositas, jeweils ausgewählt aus der Gruppe SEQ 1 - 26 und / oder SEQ 1a - 26a oder jeweils eine dafür kodierende cDNA oder jeweils einer Teilsequenz oder Fragment davon.

16. Target zur Behandlung und Therapie von Adipositas, jeweils ausgewählt aus der Gruppe SEQ 1 - 26 und / oder SEQ 1a - 26a oder jeweils eine dafür kodierende cDNA oder jeweils einer Teilsequenz oder Fragment davon.

17. Verwendung einer Markersequenz eines Peptids ausgewählt aus der Gruppe aus der Gruppe SEQ 1 - 26 und / oder SEQ 1a - 26a oder jeweils eine dafür kodierende cDNA oder jeweils einer Teilsequenz oder Fragment davon als Affinitätsmaterial zur Durchführung einer Apherese oder Blutwäsche für Patienten mit Adipositas.
